# EUROPÄISCHE PATENTSCHRIFT

(11) **EP 4 661 956 B1**
(45) Veröffentlichungstag und Bekanntmachung des Hinweises auf die Patenterteilung: **09.12.2026**
(21) Anmeldenummer: 23728616.6
(22) Anmeldetag: 10.05.2023
(51) Int. Cl.: A61N 1/372, A61N 1/378, H02J 50/10, A61N 1/375, H02J 50/90

(54) **ELEKTRONISCHES IMPLANTAT**
ELECTRONIC IMPLANT
IMPLANT ÉLECTRONIQUE

(30) Priorität: 11.02.2023 DE 102023201135
(43) Veröffentlichungstag der Anmeldung: 17.12.2025
(73) Patentinhaber: Mehnert, Walter, 85521 Ottobrunn (DE); Theil, Thomas, 82340 Feldafing (DE); Reimann, Rainer, 82386 Oberhausen (bei Peissenberg) (DE)
(72) Erfinder: MEHNERT, Walter, 85521 Ottobrunn (DE); THEIL, Thomas, 82340 Feldafing (DE)
(74) Vertreter: Kilian Kilian & Partner mbB
(86) Internationale Anmeldenummer: PCT/EP2023/062523
(87) Internationale Veröffentlichungsnummer: WO 2024/165186

(56) Entgegenhaltungen:
- EP-A1- 2 462 982
- US-A1- 2017 202 467
- US-A1- 2021 001 131

## Beschreibung

Die Erfindung betrifft ein elektronisches Implantat zur Implantation in einen Körper eines Lebewesens und zur Überwachung einer Körperfunktion. Insbesondere betrifft die Erfindung einen elektronischen Schrittmacher, der bestimmungsgemäß in/an das menschliche Herz implantiert wird.

Ein elektronisches Implantat zur Implantation in das menschliche Herz ist aus dem Patentdokument WO 2012/013212 A1 bekannt. Das dort beschriebene Implantat kommuniziert mit einer übergeordneten Steuerungseinheit per Funk, wobei das Implantat zusammen mit der Steuerungseinheit die Funktionen eines Herzschrittmachers, eines Defibrillators und einer Einheit zur Aufzeichnung von Herzaktivitäten, wie beispielsweise eines Kardiogramms, übernimmt.

Die Steuerungseinheit versorgt das Implantat mit elektrischer Energie, damit das Implantat in seiner Funktion als Herzschrittmacher/Defibrillator Spannungsimpulse zur Stimulation des Herzens abgeben oder Körpersignale zur Aufzeichnung der Herzaktivitäten erfassen kann. Die Übertragung der elektrischen Energie erfolgt, indem die Steuerungseinheit ein elektromagnetisches Wechselfeld aussendet und das Implantat die entsprechende Energie per Induktion empfängt. Hierfür besitzt das Implantat eine Empfangsspule mit einem Kern.

Eine Lösung, wie der in der Steuerungseinheit sitzende Energiespeicher und die dortige Elektronik in das Implantat integriert werden könnten, um ein autonomes Implantat zu schaffen, offenbart die genannte Druckschrift nicht.

Ähnlichen Stand der Technik zeigt die Druckschrift WO 2012/013201 A1. Ein autonom arbeitendes Implantat zeigt auch diese nicht.

Die im Stand der Technik vorgenommene Aufteilung auf mehrere Einheiten - Steuerungseinheit und Implantat - ist deshalb gewählt, weil die dort gezeigten Komponenten der Steuerungseinheiten sich im Hinblick auf Volumen und Gewicht schlicht nicht in das Implantat integrieren lassen. Außerdem lässt die im Stand der Technik beschriebene Energieübertragung ein Aufladen eines Langzeitenergiespeichers nicht zu, weil eine Leistungsabgabe der gezeigten Spulen mit hoher Windungszahl zu gering ist, insbesondere wäre der abgegebene Strom für eine Aufladung eines Akkumulators zu gering.

Die in den Patentdokumenten beschriebene Art der Energieübertragung ist nämlich in diesem Anwendungsfeld überaus problematisch. Der Grund hierfür findet sich darin, dass man sich in diesem Anwendungsfeld gegenläufigen Effekten ausgesetzt sieht.

Allgemein wäre es wünschenswert, die Frequenz des elektromagnetischen Wechselfeldes möglichst hoch zu wählen. Das ist allerdings nicht sinnvoll, weil das Feld mit steigender Frequenz aufgrund von auftretenden Skin- und Dämpfungseffekten in den menschlichen Körper nicht ausreichend weit eindringt und das Implantat nicht erreicht und der steigende hohe Wechselstromwiderstand den Ladestrom soweit reduziert, dass eine resultierende Ladezeit für die das Implantat tragende Person nicht annehmbar wäre.

Versucht man diesem Effekt durch Verringern der Frequenz unter gleichzeitiger Erhöhung der Windungszahl der Empfangsspule zu begegnen, stößt man auf die Problematik, dass einerseits der Wechselstromwiderstand der Empfangsspule mit der Windungszahl quadratisch ansteigt, und andererseits das in der Spule durch den Ladestrom erzeugte Gegenfeld proportional mit W zunimmt. Beides verminderte die für das Laden des Energiespeichers notwendige Leistungsabgabe der Spule.

Insgesamt ist damit die Energieübertragung zur Aufladung eines Langzeitspeichers in diesem Anwendungsfeld nicht gelöst.

Weiterer Stand der Technik findet sich in US 2021/001131 A1.

Vor obigem Hintergrund ist es Aufgabe der Erfindung, ein Implantat zu schaffen, das ein geringes Volumen, geringes Gewicht und eine verbesserte Energieübertragung zulässt. Zumindest ist es Aufgabe der Erfindung, ein alternatives Implantat zu schaffen.

Diese Aufgabe(n) löst ein Implantat gemäß Patentanspruch 1. Bevorzugte Ausführungsformen sind Gegenstand der Unteransprüche.

*Das elektronische Implantat zur Implantation in einen Körper eines Lebewesens und zur Überwachung einer Körperfunktion, insbesondere der Schrittmacher zur Überwachung und Steuerung der Körperfunktion, beinhaltet:*
*einen Elektrodenabschnitt, der bestimmungsgemäß an oder in einem Körperabschnitt zu befestigen oder anzuordnen ist; und*
*ein Gehäuse, das ein Volumen V_{G} im Bereich von V_{G}* ≤ *1,5 cm³, 2 cm³, 3 cm³ oder 4cm³ umfasst und das folgende Komponenten des elektronischen Implantates aufnimmt:*
   *(i) eine mit dem Elektrodenabschnitt verbundene Elektronik, die eingerichtet ist, zumindest die Körperfunktion über den Elektrodenabschnitt zu überwachen;*
   *(ii) einen Energiespeicher zur Langzeitversorgung der Elektronik mit elektrischer Energie, der nach Entladung mit elektrischer Energie wieder aufgeladen werden kann; und*
   *(iii) einen mit dem Energiespeicher elektrisch verbundenen Energieempfangsabschnitt, der derart eingerichtet ist, dass er Energie kontaktlos - per Induktion - empfangen und an den Energiespeicher zum Wiederaufladen des Energiespeichers abgeben kann; wobei*
*der Energieempfangsabschnitt aufweist:*
   *eine Spule, die sich entlang einer Spulenachse erstreckt und eingerichtet ist, die Energie zu empfangen und an den Energiespeicher abzugeben, wenn sie von einem externen (außerhalb des Körpers) erzeugten magnetischen Wechselfeld durchsetzt wird,*
   *einen magnetisch leitenden Kern, der sich in der Spule befindet und entlang der Spulenachse verläuft, wobei die Spule bevorzugt auf und um den Kern gewickelt ist, und*
   *mindestens einen sich außerhalb der Spule befindenden magnetisch leitenden Feldkollektor, der sich in Richtung der Spulenachse an einem Ende des Kerns befindet und quer zu der Spulenachse größere Abmessungen besitzt als der Kern.*

In Bezug auf die Werte des umfassten Gehäusevolumens V_{G} ist V_{G} = 1cm³ bevorzugt ausgenommen.

Das Implantat kann prinzipiell über einen weiten Bereich in einer beliebigen Position, d.h. Ort und räumlichen Ausrichtung, implantiert werden. Grund hierfür sind die größeren Abmessungen des Feldkollektors in Bezug auf den Kern und die damit einhergehende Eigenschaft des Kerns und Feldkollektors das Magnetfeld "einzufangen", selbst wenn B-Vektor des Magnetfeldes und die Spulenachse nicht parallel zueinander sind.

Das Implantat ist beispielsweise ein Herzschrittmacher, ein Hirnschrittmacher, ein Organschrittmacher oder eine Analyseeinheit. Letztere Analyseeinheit ist beispielsweise so aufgebaut, dass sie kontinuierlich oder in bestimmten Zeitabständen Parameter, wie beispielsweise Blutdruck und/oder Blutwerte ermittelt und/oder ein Kardiogramm aufzeichnet. Ist das Implantat der genannte Herzschrittmacher, ist bevorzugt vorgesehen, vom Körper für das Herz gelieferte (körpereigene) Steuerungsimpulse zu reparieren, d.h. voll auszubilden, oder fehlende körpereigene Steuerungsimpulse zu ersetzen. Besonders bevorzugt ist das Implantat ein Einkammer-Herzschrittmacher oder ein Teil eines Mehrkammer-Herzschrittmachernetzwerks, der/das im menschlichen Herz oder am menschlichen Herz sitzt bzw. an diese Positionen implantiert ist. Das Herzschrittmachernetzwerk besitzt beispielsweise zwei oder drei über elektrische Signale verbundene Implantate, die jeweils in eine Herzkammer implantiert, dort verankert werden, und miteinander kommunizieren. Die das Herzschrittmachernetzwerk bildenden Implantate können auch in den jeweiligen Herzmuskel implantiert werden.

Der Elektrodenabschnitt beinhaltet in Abhängigkeit davon, welchen Zweck das Implantat übernimmt, eine bestimmte Anzahl von Elektroden, wobei eine der Elektroden als Masse fungiert.

Wenn das Implantat die Funktion eines der genannten Schrittmacher übernimmt, werden die Elektroden bestimmungsgemäß mit dem Körperabschnitt, beispielsweise Herz oder Gehirn, der zu stimulieren ist, verbunden oder liegen an oder in diesem an.

Allgemein können die genannten Elektroden beispielsweise Kabelelektroden sein. Insbesondere beinhaltet das Implantat in diesem Zusammenhang bevorzugt pro Kabelelektrode ein Kabel bestimmter Länge, das bestimmungsgemäß zu einem gewünschten Bereich des Körperabschnitts innerhalb des Körpers verlegt werden kann. An dem Ende des Kabels ist ein bevorzugt spiralförmiger Abschnitt zur Verankerung der Kabelelektrode in dem Bereich des Körperabschnittes ausgebildet.

Alternativ kann der Elektrodenabschnitt auch ohne Kabelelektrode(n) auskommen. In diesem Fall sind die genannten Elektroden auf einer Außenoberfläche des Implantats ausgebildet, wobei es so implantiert wird, dass die Elektroden jeweils an oder in einem Bereich des Körperabschnitts anliegen und/oder dort verankert werden können. Diese Ausgestaltung ist insbesondere dann vorteilhaft, wenn das Implantat der Herzschrittmacher oder Teil des Herzschrittmachernetzwerks ist, die jeweils vollständig in/an das Herz zu implantieren sind. Das Herzschrittmachernetzwerk beinhaltet ergo dann mehrere erfindungsgemäße Implantate mit entsprechenden Elektrodenabschnitten, die auf der Außenoberfläche der jeweiligen Einheiten freiliegen.

Weiterhin alternativ kann der Elektrodenabschnitt aus einer Kombination aus mindestens einer einzigen Kabelelektrode und aus mindestens einer einzigen auf der Außenoberfläche ausgebildeten Elektrode aufgebaut sein. In diesem Fall wird das Implantat bevorzugt an dem Körperabschnitt angeordnet, sodass die auf der Außenoberfläche gebildete Elektrode mit dem entsprechenden Bereich des Körperabschnitts in Kontakt kommt und/oder dort verankert ist. Die andere Elektrode, d.h. die Kabelelektrode, wird zu einem anderen Bereich des Körperabschnittes geführt und dort verankert bzw. befestigt.

Die Elektronik des erfindungsgemäßen Implantats ist eingerichtet, zumindest eine oder mehrere Körperfunktionen zu überwachen. Hierzu gehören beispielsweise die Funktionen der genannten Analyseeinheit, d. h. die Aufzeichnung von Daten, beispielsweise von einem Kardiogramm, Blutdruckwerten oder die Aufzeichnung von Blutwerten.

Wenn das Implantat der genannte Herzschrittmacher oder Teil des Herzschrittmachernetzwerks ist, die jeweils in/an das menschliche Herz zu implantieren sind, ist die Elektronik eingerichtet, den Herzschlag zu überwachen und basierend hierauf zu erkennen, ob der Herzschlag gesteuert werden muss. Wenn dies der Fall ist, erzeugt die Elektronik einen Stimulationsimpuls, insbesondere einen Spannungsimpuls, und gibt diesen über den Elektrodenabschnitt an den Körperabschnitt ab.

Im Hinblick auf den Aufbau und Funktionen des Herzschrittmachernetzwerks wird auf die Ausführungen in der Patentanmeldung EP 3756726 A2 verwiesen. Insbesondere werden die Absätze [0011-0028] der EP 3756726 A2 durch Bezug mit aufgenommen.

Der Energiespeicher des erfindungsgemäßen Implantats ist bevorzugt ein elektrochemischer Akkumulator, der wieder aufgeladen werden kann, insbesondere ein Lithium-Ionen Akkumulator. Der Energiespeicher dient insbesondere der Langzeitversorgung des Implantates. Der Energiespeicher ist bevorzugt derart dimensioniert, dass er das gesamte Implantat für eine Laufzeit von 0,5, 0,6, 0,7, 0,8, 0,9, 1,0, 1,1, 1,2, 1,3, 1,4, 1,5, 1,6, 1,7, 1,8, 1,9, 2,0 Jahren (Ladeintervallen) mit der elektrischen Energie versorgen kann, ohne dass eine Aufladung des Energiespeichers erforderlich ist. Beispielsweise hat der Energiespeicher eine Ladungskapazität von 200 As bis 400 As (Amperesekunden, Coulomb).

Der Energiespeicher kann eine Vielzahl von Energiespeichereinheiten aufweisen, die verteilt und getrennt voneinander an verschiedenen Positionen in dem Implantat angeordnet sind, wobei zumindest eine der oder jede der Energiespeichereinheiten bevorzugt eine elektrochemische Akkumulatoreinheit, insbesondere eine Lithium-Ionen Akkumulatoreinheit ist.

Bevorzugt beinhaltet der Energieempfangsabschnitt mindestens einen Gleichrichter und mindestens einen Glättungskondensator, die sich zwischen der Spule und dem Energiespeicher befinden. Die Spule gibt die empfangene Energie über den Gleichrichter an den Glättungskondensator ab. In diesem Zusammenhang wird der von der Spule abgegebene Lade(wechsel)strom durch den Gleichrichter gleichgerichtet und von dem Glättungskondensator dem Energiespeicher zugeführt.

Von wesentlicher Bedeutung ist der Aufbau des Energieempfangsabschnitts.

Der Energieempfangsabschnitt ist eingerichtet, die Energie per Induktion zu empfangen, wobei er hierfür eine Spule beinhaltet, die von dem externen magnetischen Wechselfeld durchsetzt wird. Die Spule erzeugt in Abhängigkeit von der Änderung des durchsetzenden magnetischen Flusses die entsprechende Ladespannung und über den Gleichrichter einen entsprechenden Ladestromfluss, der zum Wiederaufladen des Energiespeichers dient. Die Ladespannung ist damit von Frequenz und Amplitude des Magnetflusses des magnetischen Wechselfeldes proportional abhängig. Insbesondere ist der Kern und der Feldkollektor kein Element, wie bspw. ein Wieganddraht/Impulsdraht, der bei einer Magnetfeldänderung bestimmter Amplitude einen großen Barkhausen-Sprung, in Form einer über den Draht laufenden Blochwand, zeigt und deshalb unabhängig von der Frequenz des magnetischen Wechselfeldes Impulse gleicher Höhe in der Spule induziert. Im Allgemeinen besitzt das Material des Kerns als Magnetflussleiter und das Material des Feldkollektors unregelmäßig magnetisch ausgerichtete Domänen.

Um den Magnetfluss durch die Spule zu erhöhen, beinhaltet der Energieempfangsabschnitt zum einen den magnetisch leitenden Kern, der sich entlang der Spulenachse in der Spule befindet, und zum anderen den Feldkollektor.

Der Feldkollektor hat erfindungsgemäß größere Abmessungen als der Kern quer, insbesondere senkrecht, zur Spulenachse und ist in Richtung der Spulenachse an einem Ende des Kerns angeordnet.

Das Gehäuse nimmt die genannten Komponenten, d.h. die Elektronik, den Energiespeicher und den Energieempfangsabschnitt, in seinem Innenraum auf und schließt diese bevorzugt hermetisch ein. Das umfasste Volumen V_{G} des Gehäuses beträgt vorzugsweise maximal 1,5 cm³, 2 cm³, 3 cm³ oder 4 cm³, wobei 1 cm³ bevorzugt ausgenommen ist. Der Elektrodenabschnitt ist, wie oben bereits erläutert, aufgebaut.

Das erfindungsgemäße Implantat ist damit insofern ein autonom arbeitendes Implantat, das seine Funktionen eigenständig ausführt, ohne dass hierfür eine Interaktion mit einer sich außerhalb des Körpers befindenden Steuerungseinheit erforderlich ist.

*Das Implantat ist erfindungsgemäß so aufgebaut, dass der Kern mit dem mindestens einen Feldkollektor eine senkrecht zur Spulenachse stehende Magnetfeldeinfangfläche A₀ mit A₀ < =2,5*10⁻³m² besitzt, die durch A₀ = Φ_{SM}*/*B₀ definiert ist, wobei Φ_{SM} der magnetische Fluss ist, der eine in Richtung der Spulenachse liegende magnetische Längsmitte bzw. eine Querschnittsfläche an einem Ort innerhalb der Spule durchsetzt, und B₀ die externe, mittlere Flussdichte über der Magnetfeldeinfangfläche A₀; und*
*die Spule derart ausgebildet ist, dass sie bei Vorliegen des magnetischen Wechselfeldes den Ladestrom bevorzugt mit einer Stärke in einem Bereich von 20mA bis 2A erzeugt.*

Aus dem Bereich des Ladestromes ausgenommen sind bevorzugt Werte von ca. 200mA.

*Das elektronische Implantat ist bevorzugt so aufgebaut, dass die magnetische Längsmitte der Ort der Querschnittsfläche der Längsmitte der Spule ist*. Dies gilt für den Fall, dass der Energieempfangsabschnitt spiegelsymmetrisch aufgebaut ist.

Bestimmungsgemäß wird das externe elektromagnetische Wechselfeld (B₀) bevorzugt so erzeugt, dass es in Richtung der Spulenachse ausgerichtet ist, d. h. der B-Vektor in die Richtung der Spulenachse weist. Der Feldkollektor sorgt aufgrund seiner größeren Abmessungen dafür, dass das elektromagnetische Wechselfeld über eine größere Magnetfeldeinfangfläche (Feldsammelfläche) A₀ verstärkt in den Kern geleitet wird. Mit anderen Worten sorgt der Feldkollektor dafür, dass die magnetische Flussdichte innerhalb des Kerns und damit innerhalb der Spule - n*B₀ - stark ansteigt.

Die Magnetfeldeinfangfläche A₀ befindet sich entlang der Spulenachse in einem bestimmten Abstand von dem Feldkollektor und verläuft senkrecht zur Spulenachse. Sie ist größer als der Feldkollektor. Diejenigen magnetischen Feldlinien, die die Magnetfeldeinfangfläche dursetzen, treten über den Feldkollektor und den Kern ein und durchlaufen die in Richtung der Spulenachse liegende magnetische Längsmitte bzw. den Ort der bestimmten Querschnittsfläche der Spule als maximaler Fluss. Verschiebt man die Magnetfeldeinfangfläche durch die Konstruktion in Richtung des Feldkollektors, verkleinert sie sich. Verschiebt man sie hingegen virtuell in die entgegengesetzte Richtung, bleibt sie (annähernd) konstant und maximal.

Die Magnetfeldeinfangfläche A₀ ergibt sich aus dem magnetischen Fluss Φ_{SM} in der magnetischen Längsmitte bzw. dem bestimmten Ort der Querschnittsfläche der Spule als maximaler Fluss und der externen Flussdichte B₀ des externen elektromagnetischen Wechselfeldes über A₀ nach der Beziehung ${A}_{0} = \frac{ΦSM}{B 0}$.

Bezeichnet man die in Richtung der Spulenachse weisende Länge des Kerns mit I_{K}, die in Richtung der Spulenachse weisende Länge des Feldkollektors mit I_{FK} sowie den senkrecht zur Spulenachse verlaufenden Durchmesser des Feldkollektors mit D_{FK}, gilt konstruktiv in Näherung unter der Annahme kreisförmiger senkrecht zur Spulenachse verlaufender Querschnitte des Kerns und Feldkollektors$\left(IK+2IFK+DFK\right) 2 * PI / 8 < A 0 < \left(IK+2IFK+DFK\right) 2 * PI / 4 .$

Eine Querschnittsfläche des Feldkollektors senkrecht zur Spulenachse ist unter maximaler Ausnutzung des genannten Volumens des Gehäuses bevorzugt 1,5-, 2-, 3-,4-, 5-, 6-, 7-, 8-, 9-, 10-, 11-, 12-, 13-, 14-, 15-, 16-, 17-, 18-, 19-, 20-, 21-, 22-, 23-, 24-, 25-, 26-, 27-, 28-, 29-, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60-fach größer als eine Querschnittsfläche des Kerns senkrecht zur Spulenachse.

Ein Ladegerät erzeugt bevorzugt das externe elektromagnetische Wechselfeld, bevorzugt mit einer magnetischen Flussdichte von B₀ = 0,02mT, 0,04mT, 0,1mT, 0,2mT, 0,5mT, 1mT, 2mT, 3mT, 4mT, 5mT, 6mT, 7mT, 8mT, 9mT, 10mT, 11mT, 12mT, 13mT, 14mT, 15mT, 16mT, 17mT, 18mT, 19mT, oder 20mT, und einer Frequenz f = 0,5kHz, 1 kHz, 2kHz, 3kHz, 4kHz, 5kHz, 6kHz, 7kHz, 8kHz, 9kHz, 10kHz, 11kHz, 12kHz,13kHz, 14kHz, 15kHz, 16kHz, 17kHz, 18kHz, 19kHz, 20kHz, oder ab f = 21kHz in 1 kHz-Schritten bis maximal 1,5 MHz. Die magnetische Flussdichte B₀ liegt insbesondere bestimmungsgemäß in einem großen räumlichen Bereich des implantierten Implantates konstant und homogen vor, beispielsweise im Herzbereich eines Menschen, an dem sich das kleine Implantat in der Funktion des Herzschrittmachers am/im Herz befindet.

Durch den Feldkollektor findet eine Verstärkung n der magnetischen Flussdichte begrenzt durch die Sättigungsfeldstärke innerhalb des Kerns statt, wobei n >= 50, 60, 70, 80, 90, 100, 110, 120, 130, 140, 150, 160, 170, 180, 190, 200, 250, 300 ist. Damit ergibt sich (Verstärkung 300) bei einer in der Umgebung der Spule räumlich homogenen magnetischen Flussdichte von beispielsweise B₀=1mT eine innerhalb des Kerns herrschende Kernflussdichte B_{K} von 0,3T. Resultierende Werte der Ladespannung/des Stroms sind ausreichend, dem Energiespeicher ausreichend Energie zum Wiederaufladen zuzuführen, selbst wenn die Frequenz f des erzeugten magnetischen Wechselfeldes in den genannten niedrigen Bereichen liegt, beispielsweise bei 20kHz.
*Bevorzugt ist das elektronische Implantat derart ausgebildet, dass*
*der Energieempfangsabschnitt einen Gleichrichter beinhaltet,*
*die Spule eingerichtet ist, wenn sie von dem magnetischen Wechselfeld durchsetzt wird, einen durch den Gleichrichter gleichgerichteten Ladestrom zu erzeugen, der dem Energiespeicher zum Wiederaufladen zugeführt wird, und*
*die Spule derart ausgebildet ist, dass sie bei Vorliegen des magnetischen Wechselfeldes den Ladestrom mit der Stärke in einem Bereich von 20mA bis 2A erzeugt.*

*Bevorzugt ist die Spule (6) derart ausgebildet, dass sie bei Vorliegen des magnetischen Wechselfeldes mit einer Frequenz in einem Bereich von 0,5 kHz bis 1,5 MHz und des magnetischen Flusses Φ_{SM} in einem Bereich von 1*10⁻⁹ Vs bis 5*10⁻⁵ Vs, den Ladestrom mit der Stärke in einem Bereich von 20mA bis 2A erzeugt.*

Der Wert *Φ_{SM} =* 1*10⁻⁹ Vs bezieht sich auf die minimal mögliche Fläche von A₀ bei minimalem B₀ = 0,02mT und der Wert Φ_{SM} = 5*10⁻⁵ Vs bezieht sich auf die maximale Fläche A₀ =2,5*10⁻³m² bei maximalem B₀ = 20mT.

Die Spule besitzt beispielsweise W Windungen, wobei bevorzugt W = 10, 20, 30, 40, 50, 100, 200, 300, 400, 500, 600, oder 700, oder 800 ist. W ist maximal W=2000, wobei die genannten niedrigeren Werte wesentlich bevorzugter sind. Besonders bevorzugt ist W ≤ 100 und hierbei bevorzugt ungleich 50. Die durch die W Windungen gebildete Wicklung kann mehrschichtig oder bevorzugt einschichtig sein. Der die W Windungen bildende Metalldraht ist beispielsweise aus Kupfer oder bevorzugt aus dem leichteren Metall Aluminium und hat einen Drahtdurchmesser von beispielsweise 60µm, 70µm, 80µm, 90µm, 100µm, 150 µm, 200 µm bis 700 µm und einen kreis- oder rechteckigen Querschnitt. Eine Länge der Spule 6 entspricht bevorzugt der des Kerns 7, sodass Enden der Spule bevorzugt Enden des Kerns entsprechen.

Aus der Windungsanzahl W, die in die Induktivität L der Spule quadratisch eingeht, ergibt sich der Wechselstromwiderstand der Spule zu ωL, sowie aus Länge und Querschnitt des die Spule bildenden Metalldrahtes der entsprechende ohmsche Widerstand.

Durchläuft das magnetische Wechselfeld die Magnetfeldeinfangfläche A₀ des Energieempfangsabschnittes, tritt es folglich in den Feldkollektor und/oder den Kern ein und durchläuft damit die Spule, sodass die Spule den Ladestrom erzeugt.

Die Spule erzeugt aufgrund des entnommenen Ladestroms in dem genannten Bereich durch Selbstinduktion ein Gegenfeld innerhalb der Spule, das das die Spule bzw. den Kern durchsetzende externe Feld schwächt. Aus der Differenz zwischen externem Feld und Gegenfeld resultiert das den Ladestrom treibende Nutzfeld.

Die Stärke des Gegenfeldes ist proportional dem Produkt aus Ladestromstärke und Windungszahl, d.h. I_{GL}*W. Insoweit ist es wünschenswert, die Windungszahl gering zu halten. Dies führt aber zu dem gegenläufigen Effekt, dass das Nutzfeld eine geringere Spannung in der Spule induziert (EMK). Die induzierte Spannung bzw. die EMK entspricht der frequenzabhängigen Änderung des magnetischen Flusses Φ multipliziert mit der Windungszahl W (EMK=-W*dΦ/dt).

Vor diesem Hintergrund ist bei Minimierung des Implantates die Windungszahl W der Spule für eine der oben angegebenen Frequenzen des magnetischen Wechselfeldes so gewählt, dass die durch das bestimmungsgemäße Nutzfeld in der Spule induzierte Spannung zwar für das Laden des Energiespeichers ausreichend hoch ist, aber gleichzeitig bspw. wegen des Gegenfeldes möglichst niedrig ist, und der Ladestrom in dem genannten Bereich zwischen 20mA und 2A liegt.

Lässt sich das für eine bestimmte Grenzfrequenz nicht realisieren, weil die durch das Nutzfeld induzierte Spannung für das Laden des Energiespeichers zu gering ist, kann bevorzugt entweder das externe magnetische Feld in seiner Stärke (B-Feld) oder durch die Konstruktion von Kern und Feldkollektor die Magnetfeldeinfangfläche A₀ erhöht werden.

Hohe Feldstärken über große Bereiche des externen magnetischen Wechselfeldes lassen sich beispielsweise mit einem Ladegerät erzeugen, wie es in EP 4035728 A1 beschrieben ist.

Mittels eines Kompensationskondensators kann der Wechselstromwiderstand der Spule im Resonanz- oder Teilresonanzbetrieb so kompensiert werden, dass der Ladestrom in dem genannten Bereich zwischen 20mA und 2A liegt.

Der Kompensationskondensator ist ganz besonders bevorzugt in seiner Kapazität so gewählt, dass der Schwingkreis aus Spule und Kompensationskondensator sich gerade nicht in Resonanz befindet, sondern die Frequenz des externen magnetischen Wechselfeldes bis 10% über oder unter der Resonanzfrequenz des Schwingkreises liegt.

Der Energiespeicher besitzt bevorzugt einen maximalen Ladungsinhalt von 200As bis 400 As (Coulomb), um eine Langzeitversorgung des Implantates zu gewährleisten.

Dadurch, dass der Energieempfangsabschnitt eingerichtet ist, den Ladestrom in dem Bereich von 20mA und 2A zu erzeugen, kann das Implantat den Energiespeicher innerhalb einer Ladezeit von zwei Stunden und weniger, vorzugsweise ≤ 20 min oder 30 min, aufladen.

*Bevorzugt ist der Feldkollektor ein Teil des Kerns, insbesondere mit dem Kern monolithisch, aus demselben Material gebildet*. Das Material des Feldkollektors und Kerns ist bevorzugt ein homogenes magnetisches Material, beispielsweise ein ferromagnetisches oder ferrimagnetisches Material.

Der Feldkollektor kann alternativ ein gegenüber dem Kern gesondertes Element sein. Gleichermaßen kann auch bei getrennter Ausbildung das Material des Kerns und/oder des Feldkollektors ein homogenes magnetisches Material sein, beispielsweise ein ferromagnetisches oder ferrimagnetisches Material.

Beispielsweise hat der Feldkollektor bei bevorzugt zylindrischer Form in Richtung der Spulenachse eine Länge I_{FK} von bevorzugt 1mm bis 5mm.

*Weiterhin bevorzugt weist das Implantat auf:*
*einen weiteren Feldkollektor, der sich in Richtung der Spulenachse an einem anderen Ende des Kerns befindet und ebenfalls quer zu der Spulenachse größere Abmessungen besitzt als der Kern selbst.*

In diesem Fall besitzt der Energieempfangsabschnitt eine weitere gleichgroße Magnetfeldeinfangfläche auf der dem weiteren Feldkollektor zugewandten Seite. Die obigen Ausführungen zu der auf der Seite des Feldkollektors liegenden Magnetfeldeinfangfläche gelten für die weitere Magnetfeldeinfangfläche gleichermaßen.

Der Feldkollektor und/oder der weitere Feldkollektor besitzen auf ihren in Richtung der Spulenachse weisenden Enden abgerundete Kanten (bspw. Außenumfangskanten), sodass Magnetfeldkonzentrationen vermieden werden.

*Bevorzugt ist der weitere Feldkollektor ein Teil des Kerns, insbesondere mit dem Kern monolithisch, aus demselben Material gebildet.*

Wenn der Feldkollektor, der Kern und der weitere Feldkollektor monolithisch sind, ist das Material bevorzugt das bereits genannte homogene magnetische Material, beispielsweise das ferromagnetische oder ferrimagnetische Material.

Der weitere Feldkollektor kann alternativ ein gegenüber dem Kern gesondertes Element sein.

Wiederum gleichermaßen kann auch bei getrennter Ausbildung das Material des Kerns und/oder des weiteren Feldkollektors ein homogenes magnetisches Material sein, beispielsweise ein ferromagnetisches oder ferrimagnetisches Material.

Beispielsweise ist der weitere Feldkollektor bei bevorzugt zylindrischer Form in Richtung der Spulenachse bevorzugt I_{K} =1mm bis 5mm lang.

Der Kern mit dem Feldkollektor und/oder dem weiteren Feldkollektor nützt einen Innenraum eines Gehäuses des Implantates bevorzugt optimal voll aus, d.h. die Außenkontur des Implantates wird durch Kern mit Feldkollektor(en) festgelegt. Das Gehäuse nimmt alle Elemente des Implantates, nämlich die Elektronik, den Energiespeicher und den Energieempfangsabschnitt integriert auf. Lediglich der Elektrodenabschnitt durchdringt bevorzugt das Gehäuse und befindet sich außerhalb des Gehäuses. Das Gehäuse ist beispielsweise aus einem nicht-ferromagnetischen Material gebildet.

Die Spulenachse der Spule definiert bevorzugt die bestimmungsgemäße Ausrichtung des Implantates. Einerseits ist aufgrund der Fähigkeiten des Implantates, über eine große Magnetfeldeinfangfläche das magnetische Wechselfeld einzufangen, die exakte Ausrichtung des Implantates im implantierten Zustand nicht unbedingt nötig. Anderseits ist das genannte Ladegerät prinzipiell in der Lage, jeder räumlichen Richtung des Implantates mit der Richtung seines B-Vektors zu entsprechen, und damit die Ladezeit zu minimieren.

*Die Spule ist bevorzugt zwischen dem Feldkollektor und dem weiteren Feldkollektor auf und um den Kern gewickelt.*

*Der Energiespeicher des Implantates beinhaltet zumindest eine Energiespeichereinheit und bevorzugt mindestens eine weitere Energiespeichereinheit. Die eine Energiespeichereinheit und, wenn bevorzugt vorgesehen, die weitere Energiespeichereinheit ist*/*sind bevorzugt in Richtung der Spulenachse (SA) relativ zu dem Kern angeordnet.*

Beispielsweise ist/sind die Energiespeichereinheit und, wenn bevorzugt vorgesehen, die weitere Energiespeichereinheit in Richtung der Spulenachse derart relativ zu dem Kern angeordnet, dass sie sich neben dem Ende des Kerns befinden oder, wenn dort einer der Feldkollektoren angeordnet ist, sie sich auf der dem Kern abgewandten Seite des entsprechenden Feldkollektors befinden. Bevorzugt berühren sich Ende des Kerns/Feldkollektor und die entsprechende Energiespeichereinheit.

Bevorzugt sind die eine Energiespeichereinheit und die weitere Energiespeichereinheit vorgesehen, wobei eine der Energiespeichereinheiten in Richtung der Spulenachse auf einer Seite des Kerns angeordnet ist und die andere der Energiespeichereinheiten auf der anderen Seite des Kerns angeordnet ist. Anders ausgedrückt sind die Energiespeichereinheiten in Richtung der Spulenachse bevorzugt so angeordnet, dass der Kern und bevorzugt der/die Feldkollektoren sich zwischen den Energiespeichereinheiten befinden.

*Besonders bevorzugt weist der Energiespeicher des Implantates zumindest eine Energiespeichereinheit und bevorzugt mindestens eine weitere Energiespeichereinheit auf, wobei*
*die eine Energiespeichereinheit und, wenn bevorzugt vorgesehen, die weitere Energiespeichereinheit jeweils ein Gehäuse aufweist*/*aufweisen, das*/*die als der Feldkollektor und*/*oder der weitere Feldkollektor wirken.*

*Besonders bevorzugt beinhaltet der Energiespeicher des Implantats zumindest eine Energiespeichereinheit und bevorzugt mindestens eine weitere Energiespeichereinheit, wobei*
*der Feldkollektor und, wenn bevorzugt vorgesehen, der weitere Feldkollektor eine Vertiefung aufweist*/*aufweisen, in der*/*denen die eine Energiespeichereinheit und, wenn bevorzugt vorgesehen, die weitere Energiespeichereinheit aufgenommen ist*/*sind*.

In einer Schnittansicht, die einer Schnittebene entspricht, in der die Spulenachse liegt, besitzt die Vertiefung bevorzugt eine C- oder U-Form.

Besonders bevorzugt sind der Feldkollektor und der weitere Feldkollektor vorgesehen und beide besitzen bevorzugt die genannte Vertiefung, in der jeweils eine der Energiespeichereinheiten aufgenommen ist.

Die Energiespeichereinheiten sind bevorzugt so in der jeweiligen Vertiefung aufgenommen, dass sie entweder vollständig in der entsprechenden Vertiefung aufgenommen/versenkt sind oder gegenüber einer Stirnseite des entsprechenden Feldkollektors vorstehen. Im letzteren Fall ist ein Gehäuse der jeweiligen Energiespeichereinheit bevorzugt aus den folgend genannten Materialien des Kerns/der Feldkollektoren gebildet, um das äußere Magnetfeld stärker zu konzentrieren und weiter zu homogenisieren. Eine Querschnittsform des Gehäuses der jeweiligen Energiespeichereinheit ist bevorzugt passgenau mit einer Querschnittsform der Vertiefung.

*Bevorzugt ist das Implantat derart aufgebaut, dass der Energiespeicher zumindest eine Energiespeichereinheit und bevorzugt mindestens eine weitere Energiespeichereinheit aufweist; und die eine Energiespeichereinheit und, wenn bevorzugt vorgesehen, die weitere Energiespeichereinheit radial zur Spulenachse zumindest abschnittsweise um den Kern angeordnet ist*/*sind. Besonders bevorzugt umläuft der Energiespeicher die Spulenachse vollständig. Die Spule befindet sich bevorzugt zwischen Energiespeicher und Kern, wobei sie bevorzugt um Letzteren gewickelt ist.*

Diese Anordnung des Energiespeichers kann eine Alternative sein zu der im Vorhergehenden erläuterten Anordnung des Energiespeichers, bei der der Energiespeicher in Richtung der Spulenachse relativ zu dem Kern angeordnet ist.

Besitzt der Energiespeicher mehrere Energiespeichereinheiten, können die Anordnungen bevorzugt kombiniert sein. Beispielsweise ist dann eine der Energiespeichereinheiten radial zur Spulenachse um den Kern angeordnet und die andere der Energiespeichereinheiten in Richtung der Spulenachse relativ zu dem Kern, d.h. neben dem Kern, angeordnet.

*Besonders bevorzugt ist*/*sind der Kern und*/*oder der Feldkollektor und*/*oder, wenn bevorzugt vorgesehen, der weitere Feldkollektor aus einem Material mit einer hohen (materialspezifischen) relativen magnetischen Permeabilität und*/*oder einer möglichst hohen (materialspezifischen) Sättigungsflussdichte gebildet.*

Beispiele für das Material sind Ferrite, insbesondere weichmagnetische Ferrite, oder amorphe Metalle, wie beispielsweise SiFe, das auch unter dem Markennamen ARNON erhältlich ist, oder Mu-Metalle, wie beispielsweise NiFe-Legierungen.

Der Kern und/oder der Feldkollektor und/oder, wenn bevorzugt vorgesehen, der weitere Feldkollektor ist/sind beispielsweise ein Vollmaterial oder ein Schichtaufbau aus einer Vielzahl von Schichten.

Der Schichtaufbau ist bevorzugt aus einer Vielzahl dünner Schichten, wie beispielsweise dünner Folien oder dünner Bleche, gebildet, zwischen denen jeweils elektrisch isolierende Schichten angeordnet sind. Die elektrisch isolierenden Schichten können die dünnen Schichten stoffschlüssig miteinander verbinden.

Wenn der Kern den Schichtaufbau besitzt, haben die einzelnen Schichten eine Dicke von beispielsweise 0,015mm, ... , 0,025mm, ... , 0,035mm, ... , 0,050mm. Die elektrisch isolierenden Schichten können dieselben Dicken aufweisen oder dünner sein.

*Bevorzugt ist*/*sind der Kern und*/*oder der Feldkollektor und*/*oder, wenn bevorzugt vorgesehen, der weitere Feldkollektor aus einem elektrisch schlecht leitenden Material, insbesondere einem Isolator, mit einer hohen relativen magnetischen Permeabilität und einer möglichst hohen Sättigungsflussdichte gebildet.*

*Bevorzugt liegt die relative magnetische Permeabilität in einem Bereich von 100,* ... , *1000, ..., 5000, ..., 10000 und besonders bevorzugt in einem Bereich von 500, ... , 1000, ..., 1500.*

*Weiterhin bevorzugt besitzt das Material eine Sättigungsflussdichte, die möglichst hoch ist. Die Sättigungsflussdichte liegt bevorzugt in einem Bereich von größer*/*gleich 0,3, 0,4, 0,5, 0,6, 0,7, 0,8, 0,9, 1,0, 1,1, 1,2, 1,3, 1,4, 1,5, oder 1,6 Tesla, besonders bevorzugt in einem Bereich von 0,4 bis 0,7 Tesla bei Ferriten, oder ganz besonders bevorzugt in einem Bereich von 1 bis 1,5 Tesla bei amorphen Metallen, wie SiFe.*

Insbesondere ist es bevorzugt, dass die genannten Elemente (Kern und/oder Feldkollektoren) aus einem Vollmaterial gebildet sind, wenn das Material der Isolator ist, und dass die genannten Elemente (Kern und/oder Feldkollektoren) den Schichtaufbau besitzen, wenn das Material zwar schlecht aber zu einem gewissen Grad elektrisch leitend ist.

Wenn der Kern und/oder der Feldkollektor und/oder, wenn bevorzugt vorgesehen, der weitere Feldkollektor aus dem Vollmaterial gebildet ist/sind, ist das Material bevorzugt der genannte Ferrit.

Wenn hingegen der Kern und/oder der Feldkollektor und/oder, wenn bevorzugt vorgesehen, der weitere Feldkollektor den Schichtaufbau hat/haben, ist das Material der Schichten bevorzugt das amorphe Metall, wie beispielsweise das SiFe, oder das Mu-Metall, wie beispielsweise die NiFe-Legierung.

Bevorzugt ist/sind der Feldkollektor und/oder der weitere Feldkollektor ein gegenüber dem Kern gesondertes und bevorzugt aus einem anderen Material gebildetes Element.

Weil die verfügbaren Materialien für Vollmaterial nicht die hohen magnetischen Werte wie z.B. die amorphen Metalle aufweisen, die aber wiederum aufgrund der elektrischen Leitfähigkeit nicht für Vollmaterial geeignet sind, ist in besonderer Weise bevorzugt, dass der Kern den Schichtaufbau aus den geschichteten dünnen Folien oder Blechen, z.B. SiFe, und die Feldkollektoren aus Vollmaterial, wie z.B. Ferrit, aufgebaut sind. Auf diese Weise können die Feldkollektoren weitestgehend richtungsunabhängig das Feld konzentrieren und gleichzeitig der Kern ein möglichst großes Feld durch die Spule leiten bei bevorzugt minimalen Durchmesser.

Der aus den Schichten aufgebaute Kern besitzt bevorzugt an dem/den Feldkollektor(en) zugewandten Ende(n) einen quer zur Spulenachse verlaufenden Verbindungsabschnitt, der mit einer Aussparung in dem entsprechenden Feldkollektor ineinandergreift, um die Elemente miteinander zu verbinden.

Der/die Verbindungsabschnitt(e) hat/haben bevorzugt auf beiden quer zur Spulenachse verlaufenden Seiten gleiche Abmessungen, ist/sind ergo zu der Spulenachse symmetrisch. Der jeweilige Verbindungsabschnitt hat insgesamt bevorzugt die gleichen Außenabmessungen wie der entsprechende Feldkollektor und ist damit im Querschnitt größer als der Kern. Auf diese Weise wird der magnetische Übergangswiderstand zwischen dem Kern und dem/den Feldkollektor(en) gering gehalten.

Der oder die Verbindungsabschnitte des Kerns sind integraler Teil des Aufbaus bzw. besitzen den Schichtaufbau. Damit sorgen der/die Verbindungsabschnitt(e) dafür, dass das durch den/die Feldkollektoren gesammelte magnetische Wechselfeld durch die Spule geleitet wird. Die Spule sitzt hierbei bevorzugt auf einem Abschnitt des Kerns, der sich an den Verbindungsabschnitt in Richtung der Spulenachse anschließt bzw. der, wenn auf jeder Seite der Verbindungsabschnitt gebildet ist, sich zwischen den Verbindungsabschnitten befindet.

Das elektronische Implantat ist bevorzugt so aufgebaut, dass der Kern einen Hohlraum zwischen den Feldkollektoren definiert, in dem der magnetisch leitende Energiespeicher aufgenommen ist, oder der Kern ein magnetisch leitendes Gehäuse des Energiespeichers bildet, in dem die elektrochemischen Komponenten des Energiespeichers aufgenommen sind.

Gemäß der ersten genannten Alternative kann der Kern, unabhängig davon, ob er selbst aus dem monolithischem Material oder dem Schichtaufbau gebildet ist, den Hohlraum aufweisen, in den der magnetisch leitende Energiespeicher mit seinem eigenständigen, die elektrochemischen Komponenten aufnehmenden, magnetisch leitenden Gehäuse eingesetzt ist. Gemäß der zweiten genannten Alternative kann, wie genannt, der Kern, unabhängig davon, ob er selbst aus dem monolithischem Material oder dem Schichtaufbau gebildet ist, das magnetisch leitende Gehäuse des Energiespeichers bilden, in dem die elektrochemischen Komponenten aufgenommen sind.

*Bevorzugt ist das Implantat so aufgebaut, dass*
*zur Wiederaufladung des Energiespeichers bestimmungsgemäß im Bereich des implantierten Implantates das magnetische Wechselfeld mit einer Flussdichte B₀ zu erzeugen ist, wodurch eine entsprechende Ladespannung in der Spule induziert wird, die einer Änderung des im Kern gebündelten Magnetflusses des externen magnetischen Wechselfeldes entspricht und zu einem von der Spule abgegebenen und dem Energiespeicher mittelbar oder unmittelbar zugeführten Lade(wechsel)strom führt,*
*der Kern und*/*oder der Feldkollektor und*/*oder, wenn bevorzugt vorgesehen, der weitere Feldkollektor aus dem Material gebildet ist*/*sind, das die hohe Sättigungsflussdichte aufweist, und*
*eine Geometrie des Kerns (7) und*/*oder des Feldkollektors und*/*oder, wenn bevorzugt vorgesehen, des weiteren Feldkollektors derart gewählt ist, dass eine Kernflussdichte B_{K}, die sich im Kern der Spule aus der vervielfachten Flussdichte (n*B₀) vermindert um ein durch den Lade(wechsel)strom erzeugtes Gegenfeld ergibt, im Bereich, insbesondere plus*/*minus 1%, 2%, 3%, 4%, 5% oder 10%,* ... *, 20% der (materialspezifischen) Sättigungsflussdichte liegt, um das Implantat räumlich zu verkleinern (minimieren)*.

Die magnetische Flussdichte B₀ kann die bereits erwähnten Werte aufweisen, insbesondere im Bereich von 20µT bis 20mT. Diese magnetische Flussdichte B₀ führt zu einem ausreichend starken Magnetfluss des magnetischen Wechselfeldes innerhalb des Kerns, weil der/die Feldkollektor(en) das Feld entsprechend stark bündeln. Erfindungsgemäß wird das Material bevorzugt so gewählt, dass das um das Gegenfeld geschwächte im Kern herrschende Feld nahe der Sättigungsflussdichte oder in dem genannten Bereich liegt.

Durch diese Ausgestaltung wird das extern erzeugte magnetische Wechselfeld hinsichtlich Baugröße des Kerns/Feldkollektor(en) optimal ausgenutzt.

Der Ladestrom wird von der Spule dem Energiespeicher bevorzugt über eine Ladeelektronik, die den mindestens den Gleichrichter und mindestens den Glättungskondensator aufweist, zugeführt.

*Das elektronische Implantat ist bevorzugt ein elektronischer Schrittmacher, insbesondere ein Herzschrittmacher, und*
*die mit dem Elektrodenabschnitt verbundene Elektronik ist bevorzugt eingerichtet, die Körperfunktion über den Elektrodenabschnitt zu überwachen und einen Impuls, insbesondere einen Spannungsimpuls, zu erzeugen und diesen über den Elektrodenabschnitt an den Körperabschnitt zur Steuerung der Körperfunktion abzugeben.*

Unter der an verschiedenen obigen Stellen erwähnten Sättigungsflussdichte ist der dem Material spezifische Flussdichtebereich gemeint, bei dem die entsprechende Magnetisierungskennlinie (B-H-Kennlinie) einen Knickbereich bzw. Übergangsbereich besitzt, unterhalb dessen die Magnetisierungskennlinie im Wesentlichen linear verläuft und oberhalb dessen die Magnetisierungskennlinie mit geringerer Steigung (nämlich mit µ₀) verläuft. Bevorzugt ist unter der Sättigungsflussdichte diejenige Flussdichte gemeint, bei der - bei weiterer Erhöhung der Feldstärke H des wirkenden magnetischen Wechselfeldes - die Polarisation des Materials nicht mehr weiter ansteigt.

Wie im Vorhergehenden bereits erwähnt sollte bei Aufladung des Energiespeichers der entsprechende Feldvektor (B-Vektor) des elektromagnetischen Wechselfeldes bevorzugt in Richtung der eine Ausrichtung des Implantates im Körper definierenden Spulenachse weisen und die Spule, Kern und Feldkollektor(en) durchsetzen. Dies führt zu bestmöglicher Induktion in Bezug auf induzierte Ladespannung und hieraus resultierenden Ladeströmen. Der Feldkollektor und/oder der weitere Feldkollektor haben signifikante Wirkung, da sie die Aufladung des Energiespeichers in weiten Bereichen unempfindlich gegenüber einer Verkippung/Fehlausrichtung zwischen Feldvektor (B-Vektor) und Spulenachse machen.

Bevorzugt besitzt die Elektronik des Implantats noch eine Kommunikationseinheit, über die sie mit der Außenwelt (außerhalb des Körpers des Lebewesens) kommunizieren kann, beispielsweise zur Übertragung von Einstellungsdaten, Einstellungsbefehlen, Analysedaten und/oder Informationsdaten, die den Ladezustand des Energiespeichers angeben. Für die Speicherung der Daten zwischen den Kommunikationen ist bevorzugt ein Datenspeicher vorgesehen.

*Die Elektronik ist bevorzugt eingerichtet, Informationen zur räumlichen Anpassung einer Ausrichtung des magnetischen Wechselfeldes an die Spulenachse zu liefern, wodurch das Implantat in einer beliebigen räumlichen Orientierung implantierbar ist.*

Beispielsweise ist die mit dem Elektrodenabschnitt verbundene Elektronik eingerichtet, Informationen zu generieren zur bevorzugt automatischen - vorzugsweise parallelen - Ausrichtung der Spulenachse des Ladegerätes zur Spulenachse des Implantates.

Insbesondere ist die Elektronik bevorzugt eingerichtet, die Informationen zur automatischen Korrektur der Abweichung zwischen der räumlichen Ausrichtung eines Vektors des durch das Ladegerät erzeugten magnetischen Wechselfeldes und der Spulenachse des Implantates zu generieren und bevorzugt an das Ladegerät zu senden, wodurch das Implantat beliebig implantierbar wird. Das magnetische Wechselfeld erzeugt beispielsweise eine Ladespule des Ladegerätes, innerhalb derer sich der Körper des Patienten bei Aufladung befindet.

*Bevorzugt ist gemäß (IV) die Elektronik eingerichtet,*
*(i) ein Startsignal zum Starten der Anpassung der Ausrichtung des magnetischen Wechselfeldes an das Ladegerät zu senden oder von diesem zu empfangen, und*
*(ii) anschließend Ladeinformationen, die eine Stärke des Ladestroms angeben*/*widerspiegeln, als die Informationen an das Ladegerät auszusenden*.

Die Elektronik kann die Informationen bevorzugt durch Messen oder Verarbeiten generieren, wobei die Informationen beispielsweise Werte über Amplituden und/oder Gradienten von induzierter Spannung und/oder Ladestromstärke beinhalten.

Die generierten Informationen bilden damit die Basis, dass das Implantat an/in völlig beliebiger Position, d.h. Ort und räumlicher Ausrichtung, in den Körper des Lebewesens implantiert werden kann.

Insbesondere ist die Elektronik bevorzugt eingerichtet, den Gradienten und/oder die Amplitude der induzierten Ladespannung und/oder des durch die induzierte Ladespannung getriebenen Ladestroms zu detektieren, und ein Signal zu generieren, das die Informationen über den Gradienten und/oder die Amplitude beinhaltet.

Die Kommunikationseinheit versendet dieses Signal - beispielsweise in bestimmten Intervallen - an die Außenwelt, wobei eine übergeordnete, das Signal empfangende Einheit, wie beispielsweise ein Ladegerät (bevorzugt gemäß EP 4035728 A1), aus der Information auf die beliebige Lage und Ausrichtung des Implantates schließen kann. Mit Kenntnis der Lage und Ausrichtung des Implantates kann die übergeordnete Einheit, wie bspw. das Ladegeräte den B-Feldvektor des magnetischen Wechselfeldes zur Optimierung der Aufladung entsprechend ausrichten.

Hervorzuheben ist hierbei, dass die anfängliche Ausrichtung des B-Feldvektors beliebige Richtungen im Raum einnehmen kann, weil eine anschließende Anpassung an die Lage und Ausrichtung des Implantates möglich ist. Auch folgt hieraus, dass bei Implantation des Implantates keine Beachtung der resultierenden Lage und Ausrichtung des Implantates geschenkt werden muss.

Bevorzugt ist die Elektronik alternativ oder zusätzlich eingerichtet,
(i) ein Startsignal zum Starten der Anpassung der Ausrichtung des - an einem Ort und mit einer bestimmten Richtung vorliegenden Vektors des - externen magnetischen Wechselfeldes an das Ladegerät zu senden oder von diesem zu empfangen, worauf das Ladegerät die Ausrichtung des magnetischen Wechselfelds gemäß einer (auch) in der Elektronik gespeicherten Bewegungsfunktion verändert, und
(ii) nach Durchlaufen der Bewegungsfunktion Lageinformationen an das Ladegerät zu senden, die angeben, wie die Spulenachse räumlich ausgerichtet ist.

Die Lageinformationen sind beispielsweise Vektordaten, die die Ausrichtung der Spulenachse im Raum bevorzugt in vorgegebenen Toleranzen angeben.

Die Bewegungsfunktion beinhaltet beispielsweise eine festgelegte Startausrichtung des magnetischen Wechselfeldes, beispielsweise parallel zu einer Längsachse einer Liege, auf der sich der Körper befindet. Ausgehend von dieser Startausrichtung verändert das Ladegerät die Ausrichtung des Wechselfeldes bzw. seines B-Vektors gemäß der festgelegten Bewegungsfunktion.

Das Implantat kann währenddessen eine Veränderung des Ladegleichstromes detektieren und nach Durchlaufen der Bewegungsfunktion mittels der Zeit ermitteln, wann der Ladegleichstrom maximal war und welche Ausrichtung das Wechselfeld zu diesem Zeitpunkt hatte. Die entsprechende Ausrichtung gibt das Implantat als die Lageinformationen aus.

Das Ladegerät kann die im Vorhergehenden angesprochene Ausrichtung des Wechselfeldes gemäß folgenden Optionen vornehmen:
- Das Ladegerät kann eine Körperhalterung (bspw. Stuhl oder Liege), auf der sich der Körper befindet, bevorzugt um drei orthogonale Achsen drehen und/oder linear versetzen; und/oder
- Das Ladegerät kann eine Ladespule, die das magnetische Wechselfeld erzeugt, bevorzugt um drei orthogonale Achsen drehen und/oder linear versetzen; und/oder
- Das Ladegerät kann das magnetische Wechselfeld, das bevorzugt durch eine Vielzahl von Ladespulen erzeugt werden, dadurch ausrichten, dass Betriebsparameter der Ladespulen verändert werden und sich individuelle magnetische Wechselfelder der einzelnen Ladespulen zu dem magnetischen Wechselfeld mit einer bestimmten Ausrichtung überlagern.

Besonders bevorzugt ist die Elektronik eingerichtet ist,
(i) einen Abschluss der räumlichen Anpassung der Ausrichtung des magnetischen Wechselfeldes zu erkennen oder von dem Ladegerät signalisiert zu bekommen, und
(ii) anschließend auf Basis des momentanen (resultierenden) Ladestroms dem Ladegerät ein Feldänderungssignal zu senden, das dem Ladegerät signalisiert, Frequenz und/oder Amplitude des magnetischen Wechselfeldes zu erhöhen oder zu vermindern.

Durch diese Ausgestaltung kann bei weitgehend optimal an die Spulenachse angepasstem Wechselfeld der Ladestrom erhöht/vermindert werden, beispielsweise optimal konstant gehalten werden, weil dadurch die verschiedenen Werte von Dämpfung des Magnetfeldes im Körper keine Rolle mehr spielen.

Besonders bevorzugt ist das erfindungsgemäße Implantat, insbesondere die bevorzugten Varianten des Energieempfangsabschnittes so ausgebildet und dimensioniert, dass bei paralleler Ausrichtung von B-Feldvektor und Spulenachse und bei einer magnetischen Flussdichte B₀ von 0,02mT bis 1mT des externen magnetischen Wechselfeldes sich bei genanntem maximalen A₀ ein mittlerer, magnetischer Fluss von 5*10⁻⁸ bis 2,5*10⁻⁶ Vs (Weber) im Kern einstellt, wobei der magnetische Fluss sich auf den unbelasteten Fall ohne Gegenfeld bezieht.

Für den Fall, dass der Wechselstromwiderstand (ωL) der Spule den ohmschen Widerstand R der Spule deutlich übersteigt, ist, wie bereits erwähnt, bevorzugt, dass die Elektronik einen zusätzlichen Kompensationskondensator besitzt, und das magnetische Wechselfeld bei der Frequenz erzeugt wird, die sich aus den Werten der Spule, des Kompensationskondensators, des ohmschen Widerstandes und der Last ergibt, also im Wesentlichen der ohmsche Widerstand R die Größe/Stärke des Ladestroms begrenzt.

Letztendlich ist der Aufbau des Implantates bei einer vorbestimmten Baugröße des Implantates, die durch das Volumen des Gehäuses gegeben ist, z.B. zylinderförmiges Gehäuse, mit einer Spule mit den W Windungen und einem Windungsquerschnitt A und einem externen Magnetfeld B₀ bevorzugt in Bezug auf den erzielten Ladestrom optimiert. Im Fall von (ωL >> R) gilt in erster Näherung, dass der Ladestrom proportional zu dem Verhältnis des Magnetflusses durch die Spule zur Induktivität der Spule (Φ/L) ist, im Resonanzfall (Φ/R). Deshalb ist die Summe der Abmessungen der Feldkollektoren und der Länge des Kerns in Richtung der Spulenachse mit dem Durchmesser der Feldkollektoren ein wichtiges Maß für die Höhe des entnehmbaren Ladestromes. Die genannten Parameter beeinflussen nämlich sowohl den Magnetfluss im Kern als auch die Induktivität der Spule.

Bei dem erfindungsgemäßen Implantat ist das konstruktive Verhältnis bevorzugt so realisiert, dass der Ladestrom sein Maximum erreicht oder maximal 10% darunterliegt. Hervorzuheben ist in Zusammenhang mit dem erzielten Ladestrom, dass der ohmsche Widerstand R der Spule bei gleichzeitig hoher Feldstärke im Kern sehr gering gehalten werden kann, im Vergleich zu einem Kern mit konstantem und den Feldkollektoren entsprechendem Durchmesser. Der geringere ohmsche Widerstand erzeugt geringere Verluste und hat damit eine wesentlich geringere Wärmentwicklung zur Folge. Aufgrund der vorgesehenen Anordnung des Implantats im menschlichen Körper, z.B. Herz, Hirn, Gewebe, Gefäß oder Organ, ist dies ein ganz wesentlicher Faktor.

Aus den Erläuterungen des erfindungsgemäßen Implantates und seinen bevorzugten Merkmalen kann Folgendes abgeleitet werden:
Die beschriebene Konstruktion des Implantates, insbesondere des Energieempfangsabschnittes, eröffnet die signifikante Möglichkeit durch viele veränderbare Parameter ein Optimum für den jeweiligen Anwendungsfall des Implantates beispielsweise als Herzschrittmacher, Hirnschrittmacher, Organschrittmacher oder Analyseeinheit zu finden. Dieses Optimum lässt sich finden, indem das im Kern resultierende Magnetfeld (Nutzfeld) maximiert wird, das Gewicht, die Verluste und insbesondere die Windungszahl W der Spule minimiert werden und das Volumen des Implantates in erster Näherung durch die Abmessungen der magnetischen Komponenten vorgegeben wird.

Bei einer gewünschten geringen Größe und Gewicht und einer autonomen Laufzeit des Implantates von beispielsweise einem Jahr und einer Ladezeit von beispielsweise kleiner als 30 Minuten ist bei der erläuterten Konstruktion des Implantates, insbesondere des Energieempfangsabschnittes, eine Optimierung hinsichtlich des Volumens und Gewichts in erster Näherung durch ein möglichst großes externes durch die Medizin beschränktes Magnetfeld (< 1mT), in zweiter Näherung durch die bei der Aufladung auftretenden Verluste bzw. Erwärmung und der Feldkonzentration im Kern gegeben.

Das erfindungsgemäße Implantat, insbesondere in seiner Ausgestaltung als akkubetriebener, autonomer Herzschrittmacher, ist universell einsetzbar und hinsichtlich Volumen und Gewicht minimiert. Hierdurch erfüllt es hohe Anforderungen und überwindet damit technologische Grenzen. Beispielsweise erlaubt die Konstruktion des Implantates mit folgenden hohen Anforderungen klarzukommen bzw. diese zu erfüllen: $Externes magnetische Wechselfeld \left(B-Feld\right) \leq 1 mT ;$ Ladekapazität: 400As; $Ladeintervall > 1 Jahr ,$ $Ladedauer < 1 Stunde ,$ $Volumen < 2 {cm}^{3} ,$ $maximal Verlustleistung < 60 mW .$

Generell gilt: je größer das externe, magnetische B-Feld, desto geringer die Ladedauer und die Baugröße.

Das maximale externe Feld (wahrscheinlich ≤ 1mT) wird vom menschlichen Körper im Zusammenspiel mit der Einwirkdauer über die Verträglichkeit (medizinisch) festgelegt.

Im Grenzfall führen 400 As kurzzeitig (10³ sec) zu einem Ladestrom von I_{GI} = 0,4 A. Das aus diesem relativ großen Ladestrom resultierende und beachtliche Gegenfeld, das proportional I_{GI} * W ist, bleibt durch geringe Windungszahlen von beispielsweise W < 50 und dem Umstand, dass die Magnetfeldeinfangfläche(n) im Verhältnis zu der geringen räumlichen Größe des Implantates ausreichend groß ist/sind trotz bzw. selbst bei geringem externen magnetischen Wechselfeld B₀, derart beherrschbar, dass das Nutzfeld (Differenz aus im Kern konzentrierten Magnetfeld und Gegenfeld) den Ladestrom treiben kann.

Hinzukommt, dass, bei dem geringen Volumen des Implantates und der Konstruktion aus Kern und Feldkollektor(en) mit einem Kerndurchmesser von dₖ ≤ 2mm, die Wicklungsverluste (Kupfer- oder Aluminiumverluste) und das Gewicht des Implantates gering sind. Der Umstand, dass bei dem erfindungsgemäßen Implantat das im Kern konzentrierte Magnetfeld weitestgehend homogen durch den Kern verläuft und die Windungszahl W gering ist, kann die Spule bevorzugt einschichtig sein, was für einen hohen Wirkungsgrad und einer Gewichtsverminderung förderlich ist.

Die genannten Anforderungen und/oder die erläuterten Effekte können selbst dann erzielt werden, wenn das externe magnetische Wechselfeld mit hohen Frequenzen erzeugt wird. Der aufgrund der kleinen Windungszahl ohnehin geringe Wechselstromwiderstand kann durch den Kompensationskondensator und den Teilresonanz- oder Resonanzbetrieb weiter reduziert werden zur Optimierung des entnehmbaren Stromes.

Die vorstehenden Ausführungen gelten für die folgende Ausführungsform gleichermaßen.

Im Folgenden wird eine bevorzugte Ausführungsform unter Bezug auf die beigefügten Figuren erläutert.
**Figur 1A** zeigt eine bevorzugte Ausführungsform des erfindungsgemäßen Implantats, wobei die Darstellung lediglich schematischer Art ist;
**Figur 1B** zeigt eine schematische Schnittansicht eines Energieempfangsabschnitts des erfindungsgemäßen Implantats;
**Figur 1C** zeigt magnetische Eigenschaften des Kerns mit Feldkollektoren in der Variante aus Figur 3A, sowie eine entsprechende Magnetfeldeinfangfläche;
**Figur 1D** zeigt einen exakten Feldverlauf über der Konstruktion aus Kern und Feldkollektoren gemäß einer durchgeführten Simulation; und
**Figur 2** zeigt eine bevorzugte Variante des Energieempfangsabschnitts des erfindungsgemäßen Implantats.
**Figuren 3A bis 3D** zeigen bevorzugte Konfigurationen des/der Feldkollektoren, wobei Kern und Feldkollektoren monolithisch ausgeführt sind und
**Figur 3E** zeigt eine alternative Variante des/der Feldkollektoren als gesonderte Elemente. In den Figuren 3A bis 3E ist lediglich die Hülle des Energiespeichers ohne elektrochemischen Inhalt gezeigt.
**Figur 4A** zeigt eine alternative Variante des Implantates, wobei Kern und Feldkollektoren identisch aufgebaut sind wie in Figuren 3A und 3B, und der Energiespeicher ein an den Kern angepasstes, ringförmiges Gehäuse aufweist. Figur 4 zeigt lediglich die Hülle des Gehäuses ohne elektrochemischen Inhalt.
**Figur 4B** zeigt wiederrum eine weitere alternative Variante des Implantates, wobei der Energiespeicher zwischen den Feldkollektoren sitzt und den Kern zumindest teilweise ausbildet.
**Figuren 5A** **und** **5B** zeigt eine weitere alternative Variante des Implantates, wobei Kern und Feldkollektoren identisch aufgebaut sind wie in Figuren 3A und 3B, und Energiespeichereinheiten um den Kern herum angeordnet sind. Figur 5B zeigt lediglich die Hülle des Gehäuses der Energiespeichereinheit ohne elektrochemischen Inhalt.

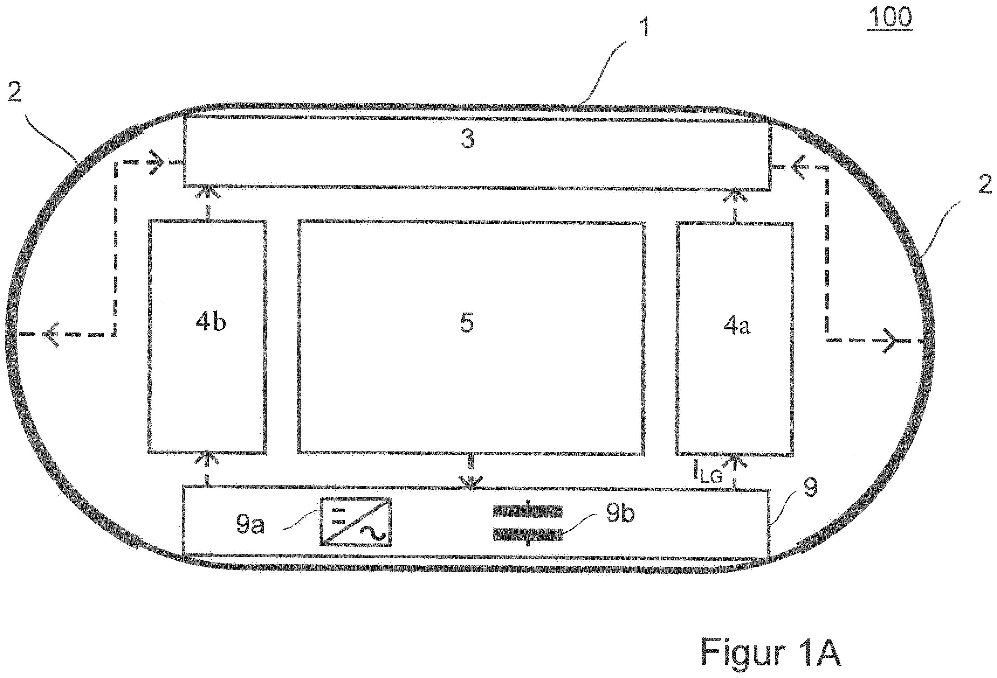

Die Figur 1A zeigt schematisch den Aufbau eines erfindungsgemäßen Implantats 100.

Das Implantat 100 wird bevorzugt vollständig in einen menschlichen Körper implantiert. Das Implantat 100 ist beispielsweise ein Herzschrittmacher, ein Hirnschrittmacher, ein Organschrittmacher oder eine Analyseeinheit. Letztere Analyseeinheit ist beispielsweise so aufgebaut, dass sie kontinuierlich oder in bestimmten Zeitabständen Parameter, wie beispielsweise Blutdruck und/oder Blutwerte ermittelt, insbesondere den das Herz stimulierenden körpereigenen Spannungsimpuls zu überwachen und ggf. zu korrigieren und im Ernstfall zu ersetzen. Besonders bevorzugt ist das Implantat ein Herzschrittmacher oder Herzschrittmachernetzwerk, der/das im menschlichen Herz oder am menschlichen Herz sitzt bzw. an diese Positionen zu implantieren ist.

Das Implantat 100 besitzt bevorzugt ein Gehäuse 1, das alle Elemente des Implantats 100 aufnimmt und bevorzugt hermetisch eingekapselt. Das Gehäuse 1 ist beispielsweise aus Titan oder Glas gebildet und umfasst ein Volumen von maximal 4 cm³, bevorzugt von kleiner/gleich 1,5 cm³ oder 2 cm³ (bevorzugt mit Ausnahme von 1 cm³)

Das Implantat 100 besitzt einen Elektrodenabschnitt mit Elektroden 2, der in Abhängigkeit davon, welchen Zweck das Implantat übernimmt bzw. welche Körperfunktion er überwachen/stimulieren soll, eine bestimmte Anzahl von Elektroden 2 aufweist. Die Elektroden 2 werden bestimmungsgemäß mit dem Körperabschnitt, beispielsweise Herz oder Gehirn, der zu überwachen und/oder zu stimulieren ist, verbunden oder liegen an diesem an.

Die Elektroden 2 können beispielsweise an ihren Enden spiralförmige Abschnitte, die in den Körperabschnitt gedreht und so verankert werden, aufweisen. Eine der Elektroden und/oder das Gehäuse, wenn leitend, können als Masseelektrode dienen.

Allgemein kann es sich bei dem erfindungsgemäßen elektronischen Schrittmacher oder dem Herzschrittmachernetzwerk um einen Herzschrittmacher gemäß einem beliebigen NBG-Code handeln.

Allgemein können die genannten Elektroden beispielsweise Kabelelektroden oder auf der Außenoberfläche freiliegende Elektrodenflächen sein.

In dem Gehäuse 1 aufgenommen sind zudem eine Elektronik 3, die eingerichtet ist, über die Elektroden 2 eine Körperfunktion zu überwachen und/oder zu stimulieren, und ein Energiespeicher mit mindestens einer, bevorzugt zwei, Energiespeichereinheiten 4a, 4b, die die Elektronik 3 mit elektrischer Energie versorgen sowie eine Ladeelektronik 9. Bevorzugt sind in der Ladeelektronik 9 ein Gleichrichter 9a und ein Glättungskondensator 9b enthalten, die einen von der Spule 6 abgegebenen Lade(wechsel)strom I_{L} gleichrichten und als I_{LG} den Energiespeichereinheiten 4 zuführen, indem der Gleichrichter 9a den von der Spule 6 abgegebenen Lade(wechsel)strom I_{L} gleichrichtet und dem Glättungskondensator 9b zuführt, und der Glättungskondensator 9b daraufhin den Strom I_{LG} an die Energiespeichereinheiten 4 weitergibt.

Die Energiespeichereinheiten, d.h. die eine Energiespeichereinheit 4a und die weitere Energiespeichereinheit 4b, sind bevorzugt jeweils wiederaufladbare, elektrochemische Akkumulatoren, beispielsweise Lithium-Ionen-Akkumulatoren, die das gesamte Implantat 100 für beispielsweise 0,5 bis 1,5 Jahre mit elektrischer Energie versorgen, bevor sie wieder aufgeladen werden müssen.

Die Energiespeichereinheiten 4a, 4b können kontaktlos wieder aufgeladen werden, unter Ausnutzung von Induktion. Hierfür besitzt das Implantat 100 einen Energieempfangsabschnitt 5, der ein wesentliches Element der Erfindung ist.

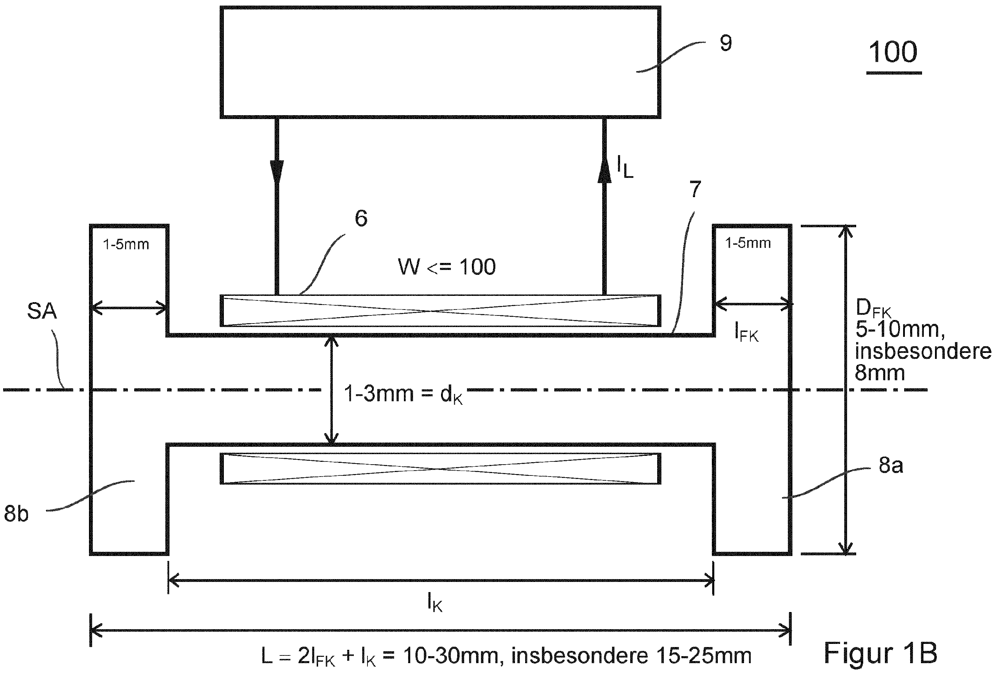

Figur 1B zeigt einen Längsschnitt des erfindungsgemäßen Energieempfangsabschnitts 5.

Dieser beinhaltet eine Spule 6 mit beispielsweise maximal 100 Windungen (W <= 100, wobei W=50 bevorzugt ausgenommen ist).

Die Spule 6 ist bevorzugt einschichtig (einlagig) auf und um einen Kern 7 gewickelt, der sich entlang einer Spulenachse SA erstreckt. Die Spulenachse SA entspricht auch einer Längsachse des Implantates 100 bzw. des Gehäuses 1.

An den jeweiligen Enden der Spule 6 bzw. des Kerns 7 befindet sich ein Feldkollektor 8a und ein weiterer Feldkollektor 8b, deren Abmessungen D_{FK} quer zur Spulenachse SA größer sind, als diejenigen des Kerns 7 innerhalb der Spule 6. Ein senkrecht zur Spulenachse SA gemessener Durchmesser dₖ des innerhalb der Spule 6 liegenden Kerns 7 aus Vollmaterial beträgt bevorzugt in Figur 1B 2mm (Millimeter) bevorzugt zwischen 1mm und 3mm. Folglich hat auch die hierauf gewickelte Spule 6 einen Innendurchmesser von etwa 2 mm. Die genannten Durchmesser des Kerns 7 bzw. Innendurchmesser der Spule 6 können in einem Bereich von 1mm bis 3mm liegen.

Eine Länge der Spule 6 entspricht bevorzugt der Länge Iₖ des Kerns 7 zwischen den Feldkollektoren 8a, 8b. Mit anderen Worten entsprechen die Enden der Spule 6 bevorzugt den Enden des Kerns 7.

Die Abmessungen des Feldkollektors 8a und des weiteren Feldkollektors 8b sind weit größer. Die entsprechenden senkrecht zur Spulenachse SA gemessenen Durchmesser D_{FK} belaufen sich zum Beispiel auf 5mm bis 10mm, bevorzugt 8mm, und haben damit beispielsweise bei 1mm Durchmesser des Kerns 7 und 8mm Durchmesser der Feldkollektoren 8a, 8b die 64fache Querschnittsfläche des Kerns 7.

Der Kern 7 und beide Feldkollektoren 8a, 8b haben bevorzugt einen kreisförmigen senkrecht zur Spulenachse SA verlaufenden Querschnitt. Alternativ kann der Querschnitt auch rechteckig, insbesondere quadratisch sein.

Aus Zusammenschau von Figur 1A und 1B ist verständlich, dass die eine Energiespeichereinheit 4a des Energiespeichers 4 in Richtung der Spulenachse SA relativ zu dem Kern 7 neben dem Feldkollektor 8a angeordnet ist, und dass die weitere Energiespeichereinheit 4b in Richtung der Spulenachse SA neben dem weiteren Feldkollektor 8b angeordnet ist. Durch diese relative Anordnung erhält das gesamte Implantat einen sehr kompakten Aufbau.

Eine Länge L des Kerns 7 mit den Feldkollektoren 8a, 8b kann 10mm bis 30mm, insbesondere 15mm bis 25mm, betragen, wobei Abmessungen I_{FK} des Feldkollektors 8a und des weiteren Feldkollektors 8b in Richtung der Spulenachse 1 bis 5mm betragen.

Die Erfindung ist nicht auf die genannten Abmessungen beschränkt. Diese sind lediglich beispielhaft.

Der Feldkollektor 8a und der weitere Feldkollektor 8b können gegenüber dem Kern 7 gesonderte Elemente sein oder integrale Bestandteile des Kerns 7 sein. Letzteres ist in Figur 1B gezeigt. Beide Feldkollektoren 8a, 8b sind monolithisch mit dem Kern aus einem einheitlichen Material gebildet. Das Material ist beispielsweise ein Ferrit. Der monolithische Aufbau ist besonders bevorzugt für den Fall, dass das Material ein Isolator oder ein zumindest elektrisch schlecht leitendes Material ist, beispielsweise ein Ferrit, weil keine oder kaum Wirbelströme auftreten.

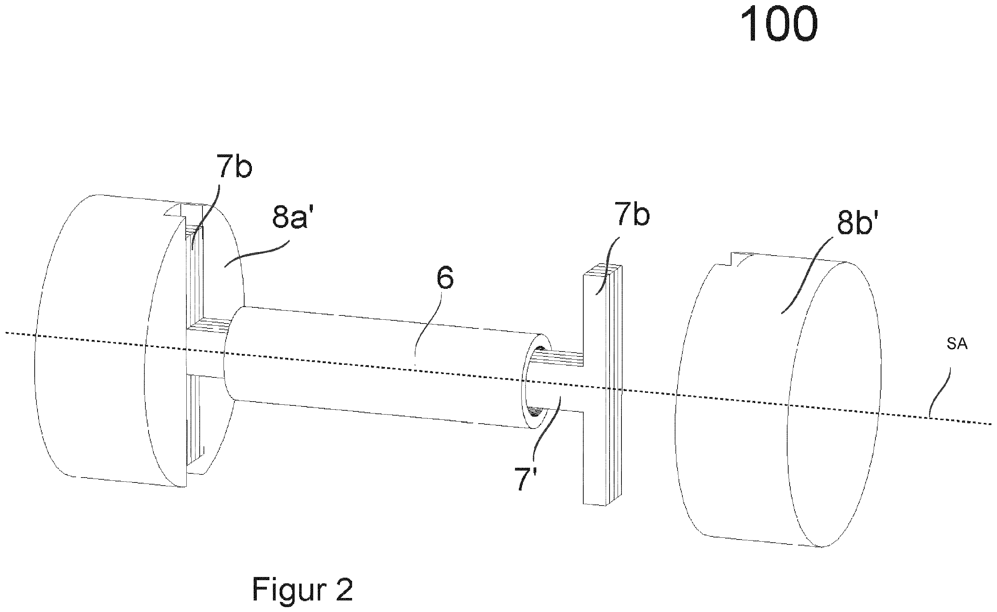

Allgemein ist der Kern und/oder die Feldkollektoren 8a, 8b aus einem Material mit einer hohen relativen magnetischen Permeabilität µᵣ (besonders bevorzugt in einem Bereich von 1000), mit einer möglichst hohen Sättigungsflussdichte (beispielsweise 0,4 bis 0,7 Tesla bei Ferriten; oder 1 bis 1,5 Tesla bei dem unter Bezug auf Figur 2 weiter unten genannten amorphen Metallen, wie SiFe) und einer möglichst geringen elektrischen Leitfähigkeit, bevorzugt einem Isolator, gebildet.

Einer der erfindungswesentlichen Gedanken ist, die Feldkollektoren 8a, 8b senkrecht zur Spulenachse SA größer als den Kern 7 dahingehend auszubilden, dass die Energiespeichereinheiten 4a, 4b des Implantats 100 durch Induktion auch bei Extrembedingungen - niedrige externe Felddichte, geringste Verlustleistung bei hoher Stromentnahme und geringes Gewicht - aufgeladen werden können.

Dies verdeutlicht folgendes Beispiel, auf das die Erfindung nicht beschränkt ist.

Wenn die Energiespeichereinheiten 4a, 4b des Implantats 100 aufgeladen werden müssen, erzeugt ein nicht gezeigtes Ladegerät ein magnetisches Wechselfeld mit einer magnetischen Flussdichte (B-Feld in beliebiger Richtung) B₀ von ungefähr 1 mT (milli Tesla), die in einem weit über das Implantat 100 reichenden Bereich homogen vorliegt. Das Feld ist insbesondere bevorzugt in Richtung der Spulenachse SA ausgerichtet (B-Vektor) und durchsetzt die Spule 6.

Das magnetische Wechselfeld ist streng genommen ein elektromagnetisches Wechselfeld. Die elektrische Komponente dieses Feldes ist allerdings von untergeordneter Bedeutung, weshalb im Rahmen dieser Anmeldung lediglich von dem magnetischen Wechselfeld gesprochen wird. Ein reines magnetisches Wechselfeld ist allerdings von der Erfindung mit umfasst.

Die Frequenz f des magnetischen Wechselfeldes liegt in einem Bereich von beispielsweise 50kHz. Bei diesen Frequenzen dringt das magnetische Wechselfeld noch gut und tief in menschliches Gewebe ein, beispielsweise bis zu dem menschlichen Herz, an dem das Implantat 100 bevorzugt sitzt.

Weil der Energieempfangsabschnitt 5 den erläuterten Kern 7 mit den Feldkollektoren 8a, 8b besitzt, bekommt der Kern 7 hierüber ausreichend Feld, damit die Spule 6 einen ausreichend hohen Lade(wechsel)strom I_{L} zur Aufladung der Energiespeichereinheiten 4a, 4b erzeugt. Aufgrund der Abmessungen senkrecht zur Spulenachse SA des Feldkollektors 8a und des weiteren Feldkollektors 8b liegt eine verstärkte Kernflussdichte B_{K} innerhalb des Kerns 7 vor. Die Kernflussdichte B_{K} übersteigt die magnetische Flussdichte B₀ zum Beispiel bis zu dem 300-fachen (B_{K}=300B₀).

Wenn die magnetische Flussdichte B₀ des von dem Ladegerät erzeugten magnetischen Wechselfeldes, die im Bereich des Implantats vorliegt, 1mT beträgt, beläuft sich folglich die Kernflussdichte B_{K} im unbelasteten Zustand auf ca. 0,3T. Vermindert wird die genannte Kernflussdichte B_{K} allerdings um das auftretende Gegenfeld innerhalb der Spule 6, das von dem Lade(wechsel)strom I_{L} herrührt.

Die sich ergebende Kernflussdichte B_{K} beläuft sich danach insgesamt auf ca. 0,2T, bei der sich eine hohe induzierte Spannung (ca. 15V in der Ausführungsform) und ein großer mittlerer Ladestrom I_{LG} (ca. 200 bis 400 mA in der Ausführungsform) ergeben. Die gezeigte Spule 6 hat eine Induktivität von kleiner 0,2mH.

Die genannten Werte erlauben die Energiespeichereinheiten 4a, 4b in ≤ 30 min mit einer Ladung von ungefähr 400 Coulomb aufzuladen.

Unabhängig von dem erläuterten Beispiel wird der Durchmesser und die Länge der Feldkollektoren 8a, 8b und die Länge des Kerns 7 umso größer gewählt, je schwächer die Flussdichte B₀ und/oder je geringer die Frequenz f des für die Aufladung erzeugten magnetischen Wechselfeldes ist. Dies im Blick werden die Abmessungen des Kerns 7, der Feldkollektoren 8a, 8b, die Parameter der Spule 6 und die restlichen Elemente so gewählt, dass das Gewicht des gesamten Implantates 100 gering ist und im Bereich von 2g bis 4g, bevorzugt 3g (Gramm), besonders bevorzugt unter 3g, liegt.

Der Ladestrom wird von der Spule 6 dem Energiespeicher bzw. den Energiespeichereinheiten 4a, 4b bevorzugt über die gezeigte Ladeelektronik 9 zugeführt.

Aus dem Vorhergehenden ist ersichtlich und verständlich, dass der Kern 7 und die Feldkollektoren 8a, 8b aus einem Material gebildet sind, das eine hohe relative magnetische Permeabilität µᵣ mit einer möglichst hohen Sättigungsflussdichte besitzt. In dem obigen Beispiel hatte der Kern 7 und die Feldkollektoren 8a, 8b, die monolithisch aus einem einheitlichen Material (Ferrit) gebildet sind, eine Sättigungsflussdichte von ca. 0,6T.

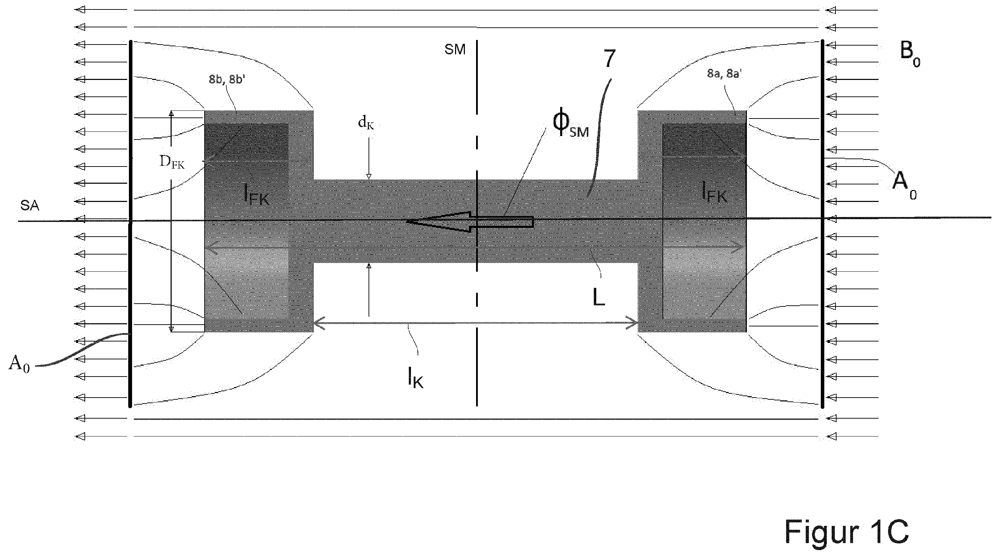

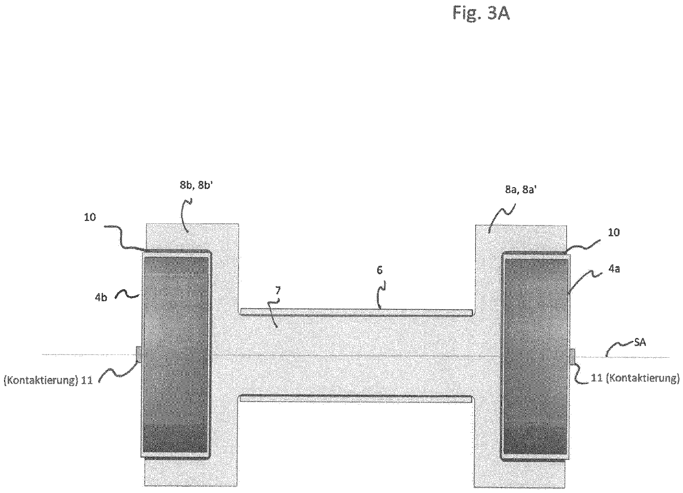

Figur 1C zeigt magnetische Eigenschaften des Kerns 7 mit Feldkollektoren 8a, 8a' und 8b, 8b' in der Variante aus Figur 3A, die im Folgenden noch beschrieben werden wird. Die Ausführungen gelten allerdings für alle gezeigten Varianten des Kerns 7 mit Feldkollektoren 8a, 8a' und 8b, 8b'.

Figur 1C zeigt schematisch eine Magnetfeldeinfangfläche A₀ für einen symmetrischen Aufbau des Energieempfangsabschnitts 5, die sich in der Figur auf der linken Seite befindet und eine weitere Magnetfeldeinfangfläche A₀, sich auf der rechten Seite befindet. Die Magnetfeldeinfangflächen ergeben sich aus den Dimensionierungen des Feldkollektors 8a, 8a', des weiteren Feldkollektors 8b, 8b' und des Kerns 7. Die gezeigten Magnetfeldeinfangflächen sind in Bezug auf die gezeigte Konstruktion maximale Magnetfeldeinfangflächen. Abmessungen der Magnetfeldeinfangflächen A₀ ergeben sich unter Verwendung der Nomenklatur aus Figur 1B in Näherung für Baugrößen des Gehäuses 1cm³ ≤ V_{G} ≤ 4cm³ und µᵣ = 10³ und symmetrischen Aufbau (µᵣ der Konstruktion aus Kern und Feldkollektoren) und d_{K} klein gegen D_{FK} aus
A₀= [(I_{K} + 2I_{FK} + D_{FK})² + ((Iₖ + 2I_{FK}) 2/PI + d_{K})² - (Iₖ - (2IFK + dK)/PI)²] PI/8, und exakt aus A₀=Φ_{SM}/B₀. Hieraus wird ersichtlich, dass A₀ eine fundamentale Größe für die Konstruktion des Implantates bzw. Herzschrittmachers ist.

Die maximalen Magnetfeldeinfangflächen A₀ befinden sich entlang der Spulenachse SA in einem bestimmten Abstand von dem jeweiligen Feldkollektor 8a, 8a', 8b, 8b' und verlaufen jeweils senkrecht zur Spulenachse SA. Sie sind jeweils wesentlich größer als der entsprechende Feldkollektor 8a, 8a', 8b, 8b'.

Das externe magnetische Wechselfeld (B₀) ist konstruktionsbedingt durch das Ladegerät nahezu homogen.

Diejenigen magnetischen Feldlinien, die die weitere Magnetfeldeinfangfläche dursetzen, treten über den weiteren Feldkollektor 8b, 8b' und den Kern 7 ein und durchlaufen die in Richtung der Spulenachse SA liegende Spule 6. Verschiebt man die Magnetfeldeinfangfläche durch die Konstruktion in Richtung des weiteren Feldkollektors 8b, 8b', verkleinert sie sich. Verschiebt man sie hingegen virtuell in die entgegengesetzte Richtung, bleibt sie konstant und maximal A₀.

Kehrt das magnetische Wechselfeld seine Polarität um, ist die Situation identisch, mit dem Unterschied, dass die die Magnetfeldeinfangfläche durchlaufenden Feldlinien in den Feldkollektor 8a, 8a' und Kern 7 eintreten und an dem weiteren Feldkollektor 8b, 8b' wieder austreten.

Die Magnetfeldeinfangflächen A₀ ergeben sich, wie erwähnt, exakt jeweils aus dem magnetischen Fluss Φ_{SM} als Maximum durch die Längsmitte der Spule 6, wenn der Energieempfangsabschnitt 5 symmetrisch aufgebaut ist, und der gemittelten externen Flussdichte B₀ des externen elektromagnetischen Wechselfeldes über A₀ nach der Beziehung ${A}_{0} = \frac{ΦSM}{B 0}$. Generell ist der Ort der Querschnittsfläche mit der größten Felddichte von der magnetischen Konstruktion abhängig.

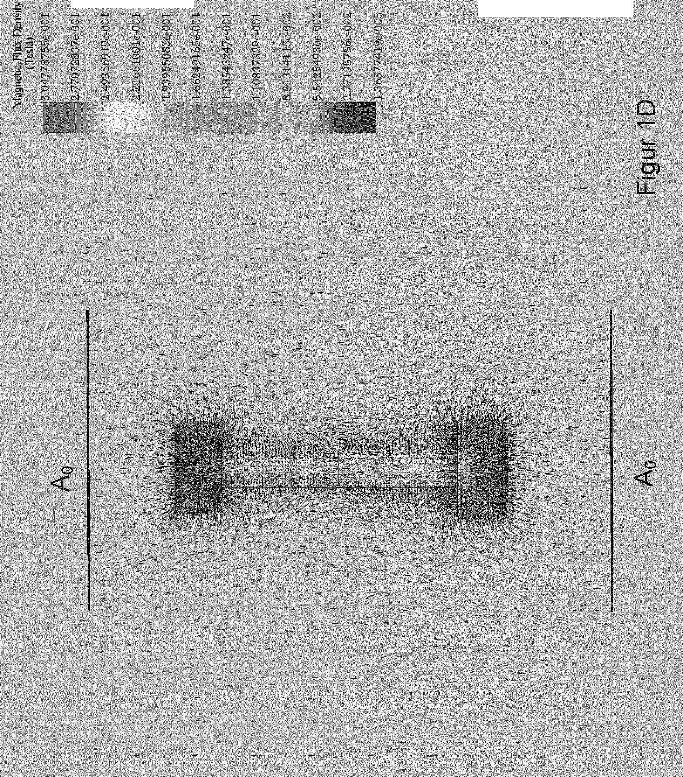

Figur 1D zeigt eine Simulation des Magnetfeldverlaufes über den Kern 7 und die Feldkollektoren 8a, 8a' und 8b, 8b', wobei ersichtlich wird, dass der magnetische Fluss im Kern 7 sehr hoch ist.

Im Vorhergehenden ist zur Erläuterung der Aufladung des Energiespeichers ein relativ starkes magnetisches Wechselfeld (1mT) mit einer relativ geringen Frequenz von 50kHz angenommen worden.

Die Erfindung ist hierauf nicht beschränkt. Die Erwägungen vor der Figurenbeschreibung hinsichtlich des Ladestromes I_{GL}, der Windungszahl W, dem magnetischen Fluss Φ_{SM} und der Frequenz des magnetischen Wechselfeldes, dem ohmschen Widerstand der Spule und einem optionalen Kompensationskondensator gelten für die Ausführungsform und alle beschriebenen Varianten der Kern-Feldkollektoren-Kombinationen gleichermaßen.

Um die Sättigungsflussdichte weiter zu erhöhen, kann ein anderes Material zum Einsatz kommen.

Besonders bevorzugt kann als alternatives Material ein amorphes Metall, beispielsweise SiFe, für den Kern 7 und/oder die Feldkollektoren 8a, 8b verwendet werden. Erhältlich auf dem Markt ist ein solches Metall zum Beispiel unter dem Markennamen ARNON.

Figur 2 zeigt im Prinzip einen bevorzugten Aufbau eines alternativen Kerns 7' inklusive alternativer Feldkollektoren 8a', 8b' des Implantates 100. Der Kern 7' hat einen Aufbau aus einer Vielzahl dünner Metallschichten, wie beispielsweise dünne Blechschichten oder dünne Metallfolien, die durch isolierende Schichten voneinander getrennt sind. Die Metallschichten sind bevorzugt aus amorphen Metall, beispielsweise SiFe, gebildet. Der Feldkollektor 8a' und der weitere Feldkollektor 8b' sind dagegen aus Vollmaterial gebildet, beispielsweise Ferrit.

Alle anderen Elemente sind zu den in Figuren 1A und 1B gezeigten identisch, weshalb auf die entsprechenden Ausführungen verwiesen wird.

Da es sich bei dem alternativen Material, dem amorphen Metall, wegen der Wirbelströme nicht um Vollmaterial handelt, haben die genannten Elemente den Schichtaufbau.

Die Dicke jeder einzelnen Schicht senkrecht zur gezeigten Spulenachse SA' liegt in der Größenordnung von 0,015mm bis 0,050mm, besonders bevorzugt bei 0,025mm. Mit diesem Aufbau werden Wirbelströme gering gehalten. Kern 7' und Feldkollektoren 8a', 8b' haben hier bevorzugt einen rechteckigen Querschnitt senkrecht zur Spulenachse SA'.

Der in Figur 2 gezeigte Aufbau des Kerns 7' aus dem genannten amorphen Metall (SiFe) besitzt eine Sättigungsflussdichte von 1T bis 1,5T und erlaubt damit zum Beispiel eine Vergrößerung der magnetischen Flussdichte B₀ des für die Aufladung erzeugten magnetischen Wechselfeldes bei der notwendigen Verkürzung der Ladezeit. Eine resultierende kleine Bauform ist dabei bedeutsam.

Da der magnetische Fluss quer zu der Schichtung aber durch die isolierenden Schichten nicht so hoch ist, wie entlang der Schichten, ist ein solcher Aufbau der Feldkollektoren nicht so wirkungsvoll wie bei Vollmaterial. In den Feldkollektoren ist aber andererseits aufgrund der größeren Volumen die Flussdichte generell niedriger, sodass ein Aufbau aus Vollmaterial besser geeignet ist. Der in Figur 2 gezeigte Aufbau entspricht daher der bevorzugten Variante, bei der der Kern 7' und die Kollektoren 8a', 8b' aus unterschiedlichen Materialien aufgebaut sind.

An den Übergängen zwischen den Materialien wird durch geeignete Konstruktionen mit entsprechender Gestaltung der Flächen darauf geachtet, dass der magnetische Widerstand gering bleibt. Eine mögliche Konstruktion ist in Figur 2 gezeigt und als Beispiel durch Verbindungsabschnitte 7a, 7b realisiert, die integraler Bestandteil des Kerns 7' und damit des Schichtaufbaus sind. Die quer zur Spulenachse SA' verlaufenden Verbindungsabschnitte 7a', 7b', greifen mit einer jeweiligen Aussparung 10 in dem entsprechenden Feldkollektor 8a', 8b' ineinander, um die gezeigten Elemente miteinander zu verbinden.

Die Spule 6 sitzt hierbei bevorzugt auf einem Abschnitt des Kerns 7', der sich an die Verbindungsabschnitte 7a, 7b in Richtung der Spulenachse SA' anschließt bzw. der sich zwischen den Verbindungsabschnitten 7a, 7b befindet.

Die Verbindungsabschnitte 7a, 7b sind jeweils symmetrisch zur Spulenachse SA' ausgebildet und haben senkrecht zur Spulenachse SA' gleiche Abmessungen wie die jeweiligen Feldkollektoren 8a', 8b'. In Richtung der Spulenachse SA' sind die Abmessungen der Verbindungsabschnitte 7a, 7b bevorzugt gleich der Tiefe der Aussparungen in den Feldkollektoren 8a', 8b', beispielsweise 1mm.

Figuren 3A bis 3D zeigen alternative Konfigurationen für die Feldkollektoren. Die Feldkollektoren sind in diesen Figuren ein monolithischer Teil des Kerns 7, wie es bereits unter Bezug auf Figur 1B erläutert wurde. Die alternativen in Figuren 3A bis 3D gezeigten Konfigurationen der Feldkollektoren sind allerdings nicht auf diese monolithische Ausgestaltung beschränkt, sondern können wie auch bei der unter Bezug auf Figur 2 erläuterten Variante als getrennte Elemente realisiert werden. Das ist bevorzugt dann der Fall, wenn Kern 7 und der/die Feldkollektor(en) 8a, 8a', 8b, 8b' aus unterschiedlichen Materialien gebildet sind. Um zu veranschaulichen, dass die der/die Feldkollektor(en) 8a, 8a', 8b, 8b' monolithisch oder als getrennte Elemente ausgeführt sein können, tragen die Feldkollektoren in Figuren 3A bis 3D die Bezugszeichen, die auch in Figuren 1B und 2 bereits benutzt wurden.

Die Figuren 3A bis 3D zeigen Schnittansichten eines Längsschnittes, wobei die Spulenachse SA der Spule 6 in den entsprechend resultierenden Schnittebenen liegt.

Wie aus den Figuren 3A bis 3D ersichtlich ist, besitzen die Feldkollektoren, d. h. der Feldkollektor 8a, 8a' und der weitere Feldkollektor 8b, 8b', jeweils eine Vertiefung 10 und besitzen bevorzugt eine einheitliche Wandstärke ausgehend von Kern 7 bis in Richtung der Spulenachse SA weisenden Stirnseite.

Die Energiespeichereinheiten 4a, 4b sind jeweils in die Vertiefungen 10 eingesetzt.

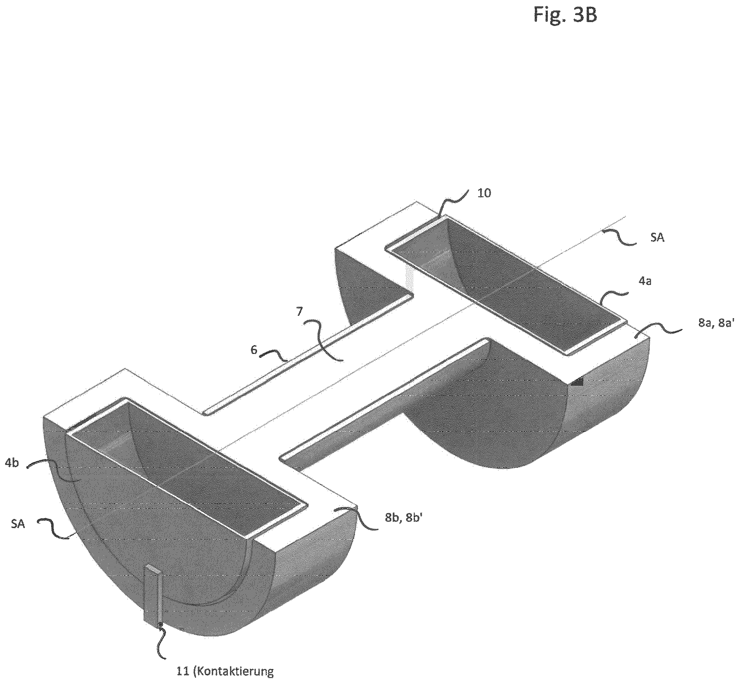

In der in Figuren 3A und 3B gezeigten Konfiguration sind die Vertiefungen 10 so dimensioniert, dass sie die jeweilige, eingesetzte Energiespeichereinheit 4a, 4b annähernd vollständig aufnehmen. Dies führt dazu, dass das Gehäuse der jeweiligen Energiespeichereinheit 4a, 4b die Führung des magnetischen Wechselfeldes kaum beeinflusst/beeinträchtigt, insbesondere verbessert, wenn Gehäuse der Energiespeicher magnetische leitend sind. Darüber hinaus wird durch die diese Maßnahme das Feld im Kern 7 weiter verstärkt und homogenisiert.

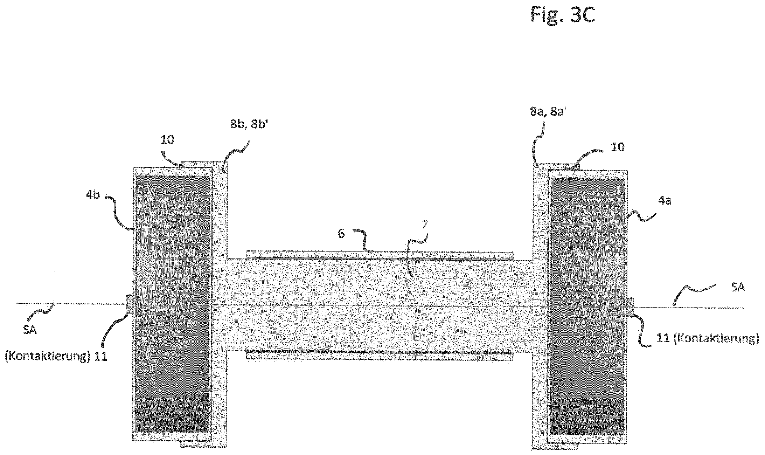

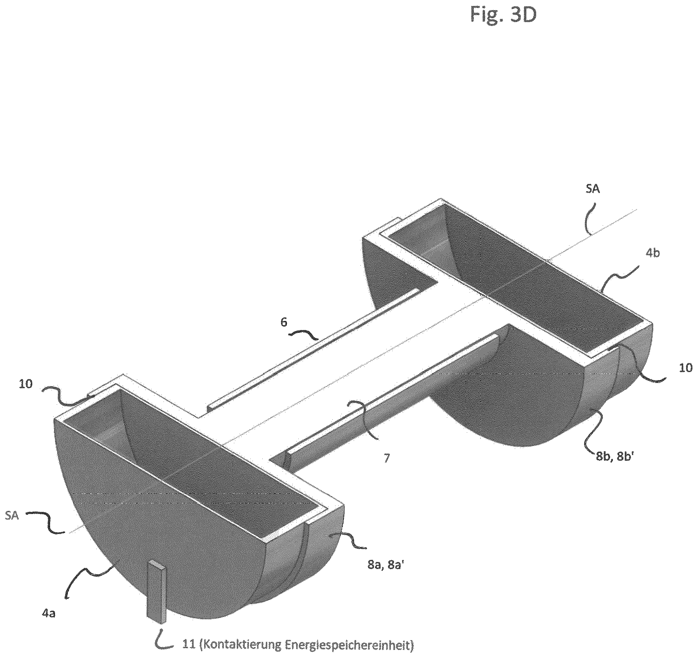

Die Konfiguration, die in Figuren 3C und 3D gezeigt ist, zeigt eine alternative Ausgestaltung der Feldkollektoren 8a, 8a', 8b, 8b'. Diese unterscheidet sich von der in Figuren 3A und 3B gezeigten dadurch, dass die Vertiefungen 10 in Richtung der Spulenachse SA geringere Abmessungen (Tiefe) haben als in Figuren 3A und 3B. Zudem sind Abschnitte des/der Feldkollektors/Feldkollektoren 8a, 8a', 8b, 8b', der in Richtung der Spulenachse SA verläuft, dünner ausgebildet, als Abschnitte, die senkrecht zur Spulenachse SA verlaufen.

Die Dimensionierung der Vertiefungen 10 gemäß Figuren 3C und 3D führt dazu, dass die Vertiefungen 10 die Energiespeichereinheiten 4a, 4b nicht vollständig aufnehmen, sondern die Gehäuse der Energiespeichereinheiten 4a, 4b in Richtung der Spulenachsen SA gegenüber dem jeweiligen Feldkollektor 8a, 8a', 8b, 8b' vorstehen.

Die Gehäuse der Energiespeichereinheiten 4a, 4b sind auch in diesem Fall bevorzugt aus einem Material ausgebildet, das die Funktion der Feldkollektoren 8a, 8a',8b, 8b' zumindest mit übernimmt. Beispielsweise sind die Gehäuse der Energiespeichereinheiten 4a, 4b aus den bereits in Zusammenhang mit dem Kern 7, 7' oder den Feldkollektoren 8a, 8a',8b, 8b' genannten Materialen (Ferrit, amorphes Metall, bspw. SiFe, oder Mu-Metall, bspw. NiFe) gebildet.

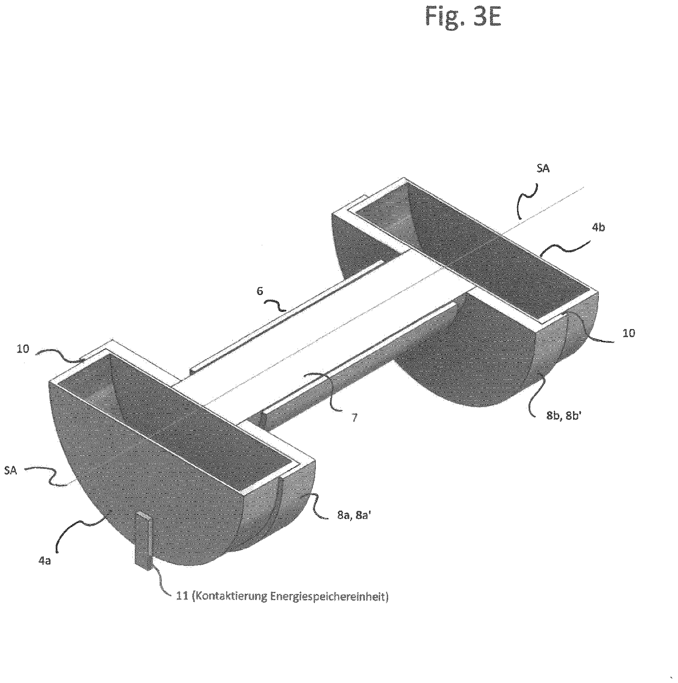

In einer anderen Variante gemäß Figur 3E können der Feldkollektor 8a, 8a' und/oder der weitere Feldkollektor 8b, 8b' ausschließlich durch das Gehäuse der jeweiligen Energiespeichereinheit 4a, 4b realisiert sein. In diesem Fall sind die Gehäuse der jeweiligen Energiespeichereinheit 4a, 4b wiederum insbesondere bevorzugt aus den genannten Materialien gebildet (Ferrit, amorphes Metall, bspw. SiFe, oder Mu-Metall, bspw. NiFe). Die Feldkollektoren 8a, 8a', 8b, 8b' sind in dieser anderen Variante keine Kollektoren im strengen Sinn, sondern formgleiche Bauteile wie in Figur 3D, die in diesem Fall nur der Halterung oder Zentrierung der Energiespeichereinheiten dienen und können auch aus einem nicht magnetischen Material, z.B. Kunststoff, gebildet sein.

Das Bezugszeichen 11 kennzeichnet eine elektrische Kontaktierung der Energiespeichereinheiten 4a, 4b.

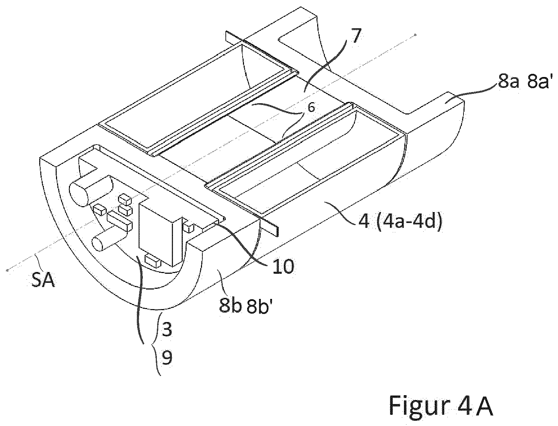

Figur 4A zeigt eine bevorzugte alternative Variante des erfindungsgemäßen Implantates 100.

Der Energieempfangsabschnitt 5 der vorliegenden Variante des Implantates 100 entspricht demjenigen aus Figur 3A und 3B, wobei die Feldkollektoren 8a,8a', 8b, 8b' zusätzlich die erläuterten Vertiefungen 10 aufweisen.

Das in Figur 4A gezeigte Implantat 100 unterscheidet sich von dem unter Bezug auf die vorstehenden Figuren 1A, 1B, 3A und 3B erläuterten Implantat 100 dadurch, dass eine Anordnung des Energiespeichers 4 und der Elektronik 3, 9 vertauscht sind.

In der in Figur 4A gezeigten Variante sind die Vertiefungen 10 so dimensioniert, dass in einer der Vertiefungen 10 die Elektronik 3 und die Ladeelektronik 9 vollständig aufgenommen ist. Alternativ können Teile der Elektronik 3 und/oder der Ladeelektronik 9 auf beide Vertiefungen 10 aufgeteilt sein. Das magnetische Wechselfeld tritt hauptsächlich an den in Richtung der Spulenachse SA weisenden Stirnseiten und den Außenseiten der Feldkollektoren 8a,8a', 8b, 8b' ein, sodass das magnetische Wechselfeld die Elektronik 3 und/oder die Ladeelektronik 9 kaum beeinflusst.

Der magnetisch nicht-leitende Energiespeicher 4 besitzt bevorzugt ein einziges Gehäuse, das an die Außenkontur bzw. Außenoberfläche der Spule 6 angepasst ist. Der Kern 7 und die Spule 6 haben bevorzugt einen senkrecht zur Spulenachse SA verlaufenden kreisförmigen Querschnitt. Demzufolge ist die Innenoberfläche bzw. die der Spule 6 zugewandte Oberfläche des Gehäuses des Energiespeichers 4 im Querschnitt (quer zur Spulenachse SA) ringförmig. Figur 4A zeigt lediglich das Gehäuse bzw. Hülle des Energiespeichers 4 und verzichtet auf die Darstellung des elektrochemischen Inhalts.

Gemäß Figur 4A ist der Energiespeicher 4 in einem einzigen, den Kern 7 vollständig umlaufenden Gehäuse aufgenommen. Beispielsweise kann das Gehäuse des Energiespeichers 4 für diese Anordnung um den Kern 7 bzw. die Spule 6 gewickelt oder gebogen werden. Das Gehäuse des Energiespeichers 4 kann bevorzugt alternativ so gewickelt oder gebogen sein, dass der Energiespeicher 4 den Kern 7 bzw. die Spule 6 nur teilweise umläuft.

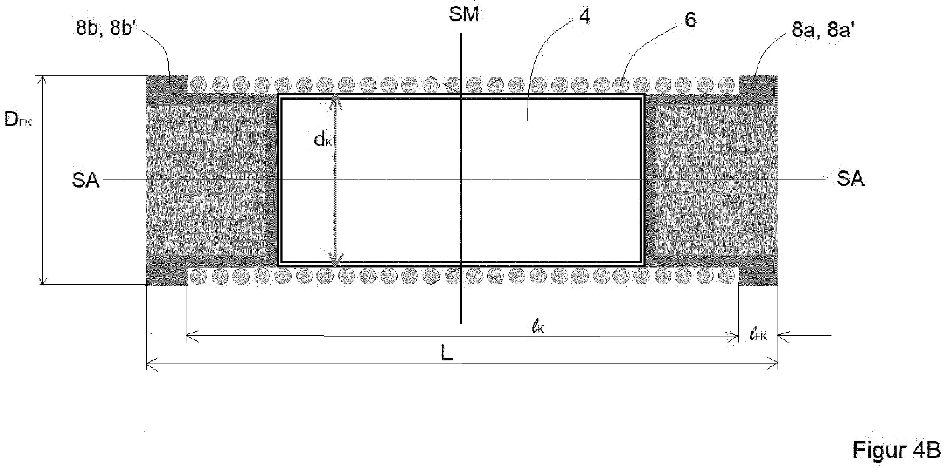

Alternativ kann - gemäß Figur 4B - das Gehäuse des Energiespeichers 4 magnetisch leitend und geschichtet sein und daher als Teil des Kerns dienen. Die Feldkollektoren 8a, 8a', 8b, 8b' besitzen Befestigungsabschnitte 10a, die sich bevorzugt in die Spule als Teil des Kerns erstrecken und bevorzugt bündig an dem Gehäuse des Energiespeichers 4 anliegen; beispielsweise sind sie dort an dem Energiespeicher stoffschlüssig befestigt. Ein Außendurchmesser des Energiespeichers 4 und ein Außendurchmesser der sich innerhalb der Spule befindenden Befestigungsabschnitte 10a der Feldkollektoren 8a, 8a', 8b, 8b' sind bevorzugt identisch.

Der Durchmesser D_{FK} der Feldkollektoren 8a, 8a', 8b, 8b' ist größer als der Durchmesser des Energiespeichers 4, um die Magnetfeldeinfangfläche A₀ zu vergrößern und das Feld zu homogenisieren. Die Feldkollektoren 8a, 8a', 8b, 8b' mit den entsprechenden Befestigungsabschnitten 10a sind bevorzugt topfartig ausgestaltet, wobei sich entsprechende darin gebildete Vertiefungen 10 im dem gezeigten Längsschnitt gesehen bevorzugt bis in die Spule 6 erstecken und beispielsweise zur Aufnahme der Elektronik 3 mit Datenspeicher, Kondensator etc. dienen.

Weiterhin alternativ kann der Energiespeicher 4 aus einer Vielzahl von Energiespeichereinheiten aufgebaut sein, die jeweils ein Gehäuse aufweisen, das einem Kreissegment um den Kern 7 aus Figur 4A herum entspricht. Bündig zusammengesetzt umlaufen die Energiespeichereinheiten den Kern 7 dann vollständig oder teilweise.

In dem Fall, dass der Energiespeicher 4 oder die Energiespeichereinheiten den Kern 7 bzw. die Spule 6 nur teilweise umlaufen, kann der freie Raum beispielsweise für Teile der Elektronik 3 und/oder der Ladeelektronik 9 und/oder einer Kommunikationseinheit zur Kommunikation mit der Außenwelt oder einem anderen Implantat genutzt werden.

Das Gehäuse des Energiespeichers 4 oder der aus den Energiespeichereinheiten zusammengesetzte Energiespeicher besitzt eine Symmetrieachse, die bevorzugt mit der Spulenachse SA identisch ist.

Bevorzugt sind die Abmessungen des Energiespeichers 4 oder der Energiespeichereinheiten radial zur Spulenachse so gewählt, dass eine der Spulenachse SA abgewandte Oberfläche bündig ist mit den Feldkollektoren 8a, 8a', 8b, 8b'.

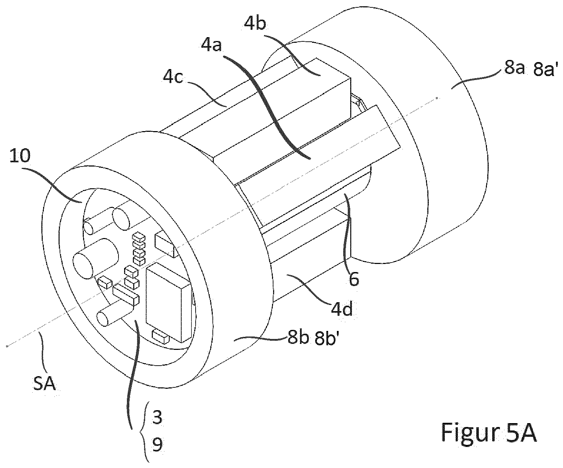

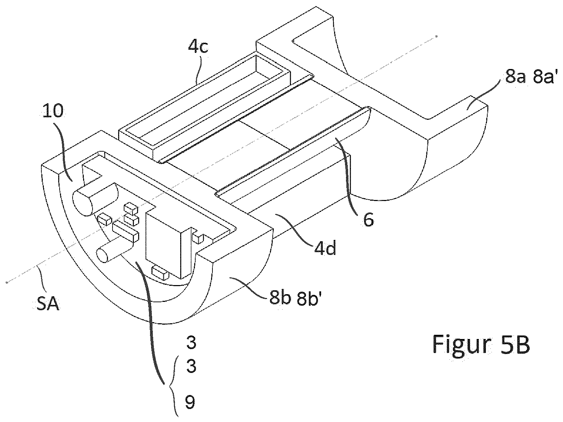

Figuren 5A und 5B zeigen eine weitere bevorzugte alternative Variante des erfindungsgemäßen Implantates 100, die sich von der aus Figur 4 lediglich durch die Ausgestaltung des Energiespeichers 4 unterscheidet.

Der Energiespeicher 4 besitzt eine Vielzahl von Energiespeichereinheiten 4a, 4b, 4c, 4d, die radial zur Spulenachse SA um den Kern 7 herum bzw. die darauf sitzende Spule 6 angeordnet sind, wobei jede der Energiespeichereinheiten 4a, 4b, 4c, 4d ein unabhängiges Gehäuse aufweist.

Die Gehäuse haben jeweils eine Quaderform, deren Längserstreckung parallel zur Spulenachse SA verläuft. Die Länge der Gehäuse in dieser Richtung, d.h. parallel zur Spulenachse SA, entspricht der Länge des Kerns 7 bzw. dem Abstand der Feldkollektor 8a, 8b.

Die Energiespeichereinheiten 4a, 4b, 4c, 4d sind in Abständen zueinander in Umfangsrichtung um den Kern 7 herum angeordnet.

Die Anzahl der Energiespeichereinheiten 4a, 4b, 4c, 4d kann so gewählt werden, dass sie zusammen den Kern 7 vollständig umlaufen, oder, wie in Figur 5A gezeigt, lediglich abschnittsweise/teilweise umlaufen.

Der in Figur 5A ersichtliche, freie Raum - der durch keine der Energiespeichereinheiten 4a, 4b, 4c, 4d besetzt ist - kann beispielsweise für Teile der Elektronik 3 und/oder der Ladeelektronik 9 und/oder einer Kommunikationseinheit zur Kommunikation mit der Außenwelt oder einem anderen Implantat genutzt werden.

Die Anordnungen gemäß Figuren 4, 5A und 5B können auch mit einem Kern 7 und Feldkollektoren 8a', 8b' (Figur 2) realisiert sein, wobei bei Bedarf in dem/den Feldkollektor 8a', 8b' auch eine Vertiefung wie in Figur 3 gebildet sein kann.

Die Varianten der Feldkollektoren 8a, 8a' und 8b, 8b' gemäß Figuren 3A bis 3E, 4, 5A und 5B haben folgenden Vorteil:
- Die Feldkollektoren 8a, 8a' und 8b, 8b' besitzen geringeres Gewicht, weil sie nicht aus einem Vollmaterial sind und dazu den Raum für die Energiespeicher oder die Elektronik bieten.
- Dabei ist die Feldreduktion von Φ_{SM} gegenüber Feldkollektoren 8a, 8a' und 8b, 8b' aus Vollmaterial aber nur im unteren %-Bereich (<3%), wenn die Ausnehmungen nicht zu tief sind (<5mm).

Das Implantat 100 kann Teil des genannten Herzschrittmachernetzwerks sein, das eine Vielzahl solcher Implantate 100 besitzt, in denen entsprechende Elemente, wie sie unter Bezug auf Figuren 1A, 1B und/oder 2 und/oder 3A bis 3D erläutert wurden, aufgenommen sind. Hinsichtlich Gewicht wiegt dann jedes dieser Implantate bevorzugt circa 3g. Die Ausführungen vor der Figurenbeschreibung gelten für die Ausführungsform entsprechend und vice versa.

## Patentansprüche

1. Elektronisches Implantat (100) zur Implantation in einen Körper eines Lebewesens und zur Überwachung einer Körperfunktion, wobei das Implantat ein Schrittmacher zur Überwachung und Steuerung der Körperfunktion ist, der bestimmungsgemäß in/an das menschliche Herz implantiert wird, wobei das Implantat aufweist:
einen Elektrodenabschnitt (2), der bestimmungsgemäß an einem Körperabschnitt zu befestigen oder anzuordnen ist; und
ein Gehäuse, das ein Volumen V_{G} im Bereich von V_{G} ≤ 4 cm³ umfasst und das folgende Komponenten des elektronischen Implantates (100) aufnimmt:
(i) eine mit dem Elektrodenabschnitt verbundene Elektronik (3), die eingerichtet ist, zumindest die Körperfunktion über den Elektrodenabschnitt (2) zu überwachen;
(ii) einen Energiespeicher (4) zur Langzeitversorgung der Elektronik (3) mit elektrischer Energie, der nach Entladung mit elektrischer Energie wieder aufgeladen werden kann; und
(iii) einen mit dem Energiespeicher (4) elektrisch verbundenen Energieempfangsabschnitt (5), der derart eingerichtet ist, dass er Energie kontaktlos empfangen und an den Energiespeicher (4) zum Wiederaufladen des Energiespeichers (4) abgeben kann;
wobei
der Energieempfangsabschnitt (5) aufweist:
eine Spule (6), die sich entlang einer Spulenachse (SA) erstreckt und eingerichtet ist, die Energie zu empfangen und an den Energiespeicher (4) abzugeben, wenn sie von einem externen magnetischen Wechselfeld durchsetzt wird,
einen magnetisch leitenden Kern (7), der sich in der Spule (6) befindet und entlang der Spulenachse (SA) verläuft, und
mindestens einen sich außerhalb der Spule (6) befindenden Feldkollektor (8a), der sich in Richtung der Spulenachse (SA) an einem Ende des Kerns (7) befindet und quer zu der Spulenachse (SA) größere Abmessungen besitzt als der Kern (7);
wobei
der Kern (7) mit dem mindestens einen Feldkollektor (8a) eine senkrecht zur Spulenachse (SA) stehende Magnetfeldeinfangfläche A₀ mit A₀ <=2,5*10⁻³m² besitzt, die durch A₀ = Φ_{SM}/B₀ definiert ist, wobei Φ_{SM} der magnetische Fluss ist, der eine in Richtung der Spulenachse (SA) liegende magnetische Längsmitte innerhalb der Spule (6) als Maximum durchsetzt, und B₀ die externe, mittlere Flussdichte über der Magnetfeldeinfangfläche A₀; und
die Spule (6) derart ausgebildet ist, dass sie bei Vorliegen des magnetischen Wechselfeldes einen Ladestrom mit der Stärke in einem Bereich von 20mA bis 2A erzeugt.

2. Elektronisches Implantat nach Patentanspruch 1, wobei
der Energieempfangsabschnitt (5) einen Gleichrichter beinhaltet,
die Spule (6) eingerichtet ist, wenn sie von dem magnetischen Wechselfeld durchsetzt wird, einen durch den Gleichrichter gleichgerichteten Ladestrom zu erzeugen, der dem Energiespeicher (4) zum Wiederaufladen zugeführt wird, und
die Spule (6) derart ausgebildet ist, dass sie bei Vorliegen des magnetischen Wechselfeldes mit einer Frequenz in einem Bereich von 0,5 kHz bis 1,5 MHz und des magnetischen Flusses Φ_{SM} in einem Bereich von 1*10⁻⁹ Vs bis 5*10⁻⁵ Vs, den Ladestrom mit der Stärke in einem Bereich von 20mA bis 2A erzeugt.

3. Implantat (100) nach einem der Patentansprüche 1 bis 2, wobei eine Windungszahl W der Spule (6) in einem Bereich von W ≤ 2000, bevorzugt von W ≤ 100, liegt.

4. Implantat (100) nach einem der Patentansprüche 1 bis 3, wobei
der Feldkollektor (8a) ein Teil des Kerns (7) ist, insbesondere mit dem Kern (7) monolithisch, aus demselben Material gebildet ist.

5. Implantat (100) nach einem der Patentansprüche 1 bis 4, weiterhin aufweisend:
einen weiteren Feldkollektor (8b), der sich in Richtung der Spulenachse (SA) an einem anderen Ende des Kerns (7) befindet und quer zu der Spulenachse (SA) größere Abmessungen besitzt als der Kern (7).

6. Implantat (100) nach Patentanspruch 5, wobei
der weitere Feldkollektor (8b) ein Teil des Kerns (7) ist, insbesondere mit dem Kern (7) monolithisch, aus demselben Material gebildet ist.

7. Implantat (100) nach einem der Patentansprüche 1 oder 5, wobei
der Feldkollektor (8a) und/oder der weitere Feldkollektor (8b) ein gegenüber dem Kern (7) gesondertes und bevorzugt aus einem anderen Material gebildetes Element ist.

8. Implantat (100) nach einem der Patentansprüche 1 bis 7, wobei
der Energiespeicher (4) zumindest eine Energiespeichereinheit (4a) und bevorzugt mindestens eine weitere Energiespeichereinheit (4b) aufweist; und
die eine Energiespeichereinheit (4a) und, wenn bevorzugt vorgesehen, die weitere Energiespeichereinheit (4b) in Richtung der Spulenachse (SA) relativ zu dem Kern angeordnet ist/sind.

9. Implantat (100) nach Patentanspruch 7, wobei
der Energiespeicher (4) zumindest eine Energiespeichereinheit (4a) und bevorzugt mindestens eine weitere Energiespeichereinheit (4b) aufweist; und
die eine Energiespeichereinheit (4a) und, wenn bevorzugt vorgesehen, die weitere Energiespeichereinheit (4b) jeweils ein Gehäuse aufweist/aufweisen, das/die als der Feldkollektor (8a) und/oder der weitere Feldkollektor (8b) wirken.

10. Implantat (100) nach einem der Patentansprüche 1 bis 9, wobei
der Energiespeicher (4) zumindest eine Energiespeichereinheit und bevorzugt mindestens eine weitere Energiespeichereinheit aufweist, und
der Feldkollektor (8a) und, wenn bevorzugt vorgesehen, der weitere Feldkollektor (8b) eine Vertiefung aufweist/aufweisen, in der/denen die eine Energiespeichereinheit (4a) und, wenn bevorzugt vorgesehen, die weitere Energiespeichereinheit (4b) aufgenommen ist/sind.

11. Implantat (100) nach einem der Patentansprüche 5 bis 10, wobei
die Spule (6) zwischen dem Feldkollektor (8a) und dem weiteren Feldkollektor (8b) auf und um den Kern (7) gewickelt ist.

12. Implantat (100) nach einem der Patentansprüche 1 bis 7 oder 11, wobei
der Energiespeicher (4) zumindest eine Energiespeichereinheit (4a) und bevorzugt mindestens eine weitere Energiespeichereinheit (4b) aufweist; und
die eine Energiespeichereinheit (4a) und, wenn bevorzugt vorgesehen, die weitere(n) Energiespeichereinheit(en) (4b, 4c, 4d) radial zur Spulenachse (SA) zumindest abschnittsweise um die Spule angeordnet ist/sind.

13. Implantat (100) nach Patentanspruch 12, wobei
der Energiespeicher (4) die Spulenachse (SA) vollständig umläuft.

14. Implantat (100) nach Patentanspruch 12 oder 13, wobei
die Energiespeichereinheit (4a, 4b, 4c, 4d) radial zur Spulenachse (SA) um die Spule (6) angeordnet ist.

15. Implantat (100) nach einem der Patentansprüche 1 bis 14, wobei der Kern (7) und/oder der Feldkollektor (8a) und/oder, wenn bevorzugt vorgesehen, der weitere Feldkollektor (8b) aus einem Material mit einer hohen relativen magnetischen Permeabilität und/oder einer möglichst hohen Sättigungsflussdichte, beispielsweise einem Ferrit, bevorzugt als Vollmaterial gebildet ist/sind.

16. Implantat (100) nach einem der Patentansprüche 1 bis 15, wobei der Kern (7) und/oder der Feldkollektor (8a) und/oder, wenn bevorzugt vorgesehen, der weitere Feldkollektor (8b) einen Aufbau hat/haben, der aus einer Vielzahl einzelner dünner Schichten gebildet ist, und
das Material dieser Schichten eine hohe relative magnetische Permeabilität und/oder eine hohe Sättigungsflussdichte hat, und bevorzugt ein amorphes Metall, beispielsweise SiFe, oder ein Mu-Metall, wie beispielsweise NiFe, ist.

17. Implantat nach Patentanspruch 16, wobei
der Feldkollektor (8a) und der weitere Feldkollektor (8b) ein gegenüber dem Kern (7) gesondertes und aus einem anderen Material gebildetes Element ist, und
der Feldkollektor (8a) und der weitere Feldkollektor (8b) aus dem Vollmaterial gebildet sind und der Kern (7) den Aufbau mit den dünnen Schichten aufweist.

18. Implantat (100) nach einem der vorangehenden Patentansprüche 1 bis 17, wobei der Energieempfangsabschnitt (5) mindestens einen Gleichrichter (9a) und mindestens einen Kondensator (9b) aufweist, die sich zwischen der Spule (6) und dem Energiespeicher (4) befinden, und
die Spule (6) die empfangene Energie über den Gleichrichter (9a) und den Kondensator (9b) an den Energiespeicher (4) abgibt.

19. Implantat (100) nach einem der vorhergehenden Patentansprüche 1 bis 18, wobei
zur Wiederaufladung des Energiespeichers (4) bestimmungsgemäß im Bereich des implantierten Implantates (100) das externe magnetische Wechselfeld mit einer Flussdichte B₀ zu erzeugen ist, wodurch eine entsprechende Ladespannung in der Spule (6) induziert wird, die zu einem von der Spule (6) abgegebenen und dem Energiespeicher (4) mittelbar oder unmittelbar zugeführten Ladestrom führt,
der Kern (7) und/oder der Feldkollektor (8a) und/oder, wenn bevorzugt vorgesehen, der weitere Feldkollektor (8b) aus einem Material gebildet ist/sind, das eine hohe Sättigungsflussdichte aufweist, und
die Geometrie des Kerns (7) und/oder des Feldkollektors (8a) und/oder, wenn bevorzugt vorgesehen, des weiteren Feldkollektors (8b) derart gewählt ist, dass eine Flussdichte B_{K}, die sich im Kern der Spule (6) aus der vervielfachten Flussdichte (n*B₀) vermindert um ein durch den Lade(wechsel)strom erzeugtes Gegenfeld ergibt, im Bereich der Sättigungsflussdichte liegt.

20. Elektronisches Implantat (100) nach einem der vorhergehenden Patentansprüche 1 bis 19, wobei das Implantat (100) ein elektronischer Schrittmacher, insbesondere ein Herzschrittmacher, ist, und
die mit dem Elektrodenabschnitt (2) verbundene Elektronik (3) eingerichtet ist, die Körperfunktion über den Elektrodenabschnitt (2) zu überwachen und einen Impuls, insbesondere einen Spannungsimpuls, zu erzeugen und diesen über den Elektrodenabschnitt (2) an den Körperabschnitt zur Steuerung der Körperfunktion, bevorzugt als Ersatz für fehlende körpereigene Impulse oder zur Korrektur verkümmerter körpereigener Impulse, abzugeben, und bevorzugt weitere Körperdaten zu messen, abzuspeichern und bevorzugt zu senden.

21. Elektronisches Implantat (100) nach einem der vorhergehenden Patentansprüche 1 bis 20, wobei bei Verwendung des externen magnetischen Wechselfeldes mit einer Flussdichte B₀ von 20µT bis 10mT sich ein mittlerer, magnetischer Fluss von 1*10⁻⁹ bis 2,5*10⁻⁵ Vs (Weber) im Kern (7) einstellt.

22. Elektronisches Implantat (100) nach einem der vorhergehenden Patentansprüche 1 bis 21, wobei
ein sich aus Induktivität der Spule (6) und der Frequenz des externen magnetischen Wechselfeldes ergebender Wechselstromwiderstand (ωL) der Spule (6) den ohmschen Widerstand der Spule (6) übersteigt,
die Elektronik einen Kompensationskondensator besitzt, und
Wechselstromwiderstand und ohmscher Widerstand für das bestimmungsgemäß zu verwendende magnetische Wechselfeld derart dimensioniert sind, dass zur Optimierung des Ladestroms die Induktivität der Spule und die Kapazität des Kompensationskondensators sich in Teilresonanz oder Resonanz befinden.

23. Elektronisches Implantat (100) nach einem der vorhergehenden Patentansprüche 1 bis 22, wobei
die mit dem Elektrodenabschnitt (2) verbundene Elektronik (3) eingerichtet ist, Informationen zu generieren zur automatischen, räumlichen, vorzugsweise parallelen, Ausrichtung der Spulenachse des Ladegerätes zur Spulenachse des Implantates als Bedingung der Implantation des Implantates in einer beliebigen räumlichen Ausrichtung in drei Achsen in den Körper des Lebenswesens.

24. Elektronisches Implantat (100) nach einem der vorhergehenden Patentansprüche 1 bis 23, wobei
die Spule (6) derart ausgebildet ist, dass sie bei Vorliegen des magnetischen Wechselfeldes den Energiespeicher, der vorzugsweise eine Ladungskapazität von 200As bis 400As besitzt, in einer Zeit von weniger einer Stunde, vorzugsweise weniger 30 min oder 20 min, auflädt.

25. Elektronisches Implantat (100) nach einem der vorhergehenden Patentansprüche 1 bis 24, wobei
ein Hohlraum zwischen den Feldkollektoren definiert ist, in dem das magnetisch leitende Gehäuse des Energiespeichers (4) als der Kern (7) oder zumindest als Teil des Kerns aufgenommen ist, in dem die elektrochemischen Komponenten des Energiespeichers (4) aufgenommen sind.

26. Elektronisches Implantat (100) nach einem der vorhergehenden Patentansprüche 1 bis 25, wobei der Energieempfangsabschnitt spiegelsymmetrisch aufgebaut ist, sodass die magnetische Längsmitte mit der Längsmitte der Spule zusammenfällt.

## Claims

1. Electronic implant (100) for implantation into a body of a living being and for monitoring a bodily function, wherein the implant is a pacemaker for monitoring and controlling the bodily function, to be implanted into/on the human heart, the implant comprising:
an electrode portion (2) that is, according to its intended purpose, to be attached to or to be arranged at a body portion; and
a housing which encases a volume V_{G} in the range of V_{G} ≤ 4cm³ and which accommodates the following components of the electronic implant (100):
(i) an electronics assembly (3) connected to the electrode portion, which is configured to monitor at least the body function via the electrode portion (2);
(ii) an energy storage (4) to long-term-supply the electronics assembly (3) with electrical energy which can be recharged with electrical energy after discharge; and
(iii) an energy receiving portion (5) electrically connected to the energy storage (4), which is configured so as to be able to receive energy without contact and to deliver the energy to the energy storage (4) for recharging the energy storage (4);
wherein
the energy receiving portion (5) comprises:
a coil (6) extending along a coil axis (SA) and being configured to receive the energy and to deliver the energy to the energy storage (4) when passed through by an external alternating magnetic field,
a magnetically conductive core (7), which is located in the coil (6) and extends along the coil axis (SA), and
at least one field collector (8a) located outside the coil (6), which is located at one end of the core (7) in the direction of the coil axis (SA) and has larger dimensions transverse to the coil axis (SA) than the core (7); wherein
the core (7) with the at least one field collector (8a) has a magnetic field collecting area A₀ perpendicular to the coil axis with A₀ <=2.5*10⁻³m² , which is defined by A₀ =Φ_{SM}/B₀ , where Φ_{SM} is the magnetic flux passing through, as maximum, a magnetic longitudinal center lying in the direction of the coil axis (SA) within the coil, and B₀ is the external mean flux density over the magnetic field collecting area A₀; and
the coil (6) is designed in such a way that it generates a charging current in the range from 20mA to 2A when the alternating magnetic field is present.

2. Electronic implant according to claim 1, wherein
the energy receiving portion comprises (5) a rectifier,
the coil is (6) configured to, when penetrated by the alternating magnetic field, to generate a charging current rectified by the rectifier, which supplies the energy storage (4) for recharging, and
the coil (6) is designed such that it generates the charging current with the strength in the range from 20mA to 2A when the alternating magnetic field with a frequency in the range from 0.5 kHz to 1.5 MHz and the magnetic flux Φ_{SM} in the range from 1*10⁻⁹ Vs to 5*10⁻⁵ Vs is present.

3. Implant (100) according to any one of claims 1 to 2, wherein a number of windings W of the coil (6) is in a range of W ≤ 2000, preferably of W ≤ 100.

4. Implant (100) according to any one of patent claims 1 to 3,
wherein
the field collector (8a) is a part of the core (7), in particular is monolithic with the core (7) and is formed from the same material.

5. Implant (100) according to any one of patent claims 1 to 4, further comprising:
a further field collector (8b), which is located in the direction of the coil axis (SA) at another end of the core (7) and has larger dimensions transverse to the coil axis (SA) than the core (7).

6. Implant (100) according to patent claim 5, wherein
the further field collector (8a) is a part of the core (7), in particular is monolithic with the core (7) and is formed from the same material.

7. Implant (100) according to one of patent claims 1 or 5, wherein
the field collector (8a) and/or the further field collector (8b) is a separate element from the core (7) and is preferably formed from a different material.

8. Implant (100) according to any one of patent claims 1 to 7,
wherein
the energy storage (4) comprises at least an energy storage unit (4a) and preferably at least a further energy storage unit (4b); and
the energy storage unit (4a) and, if preferably provided, the further energy storage unit (4b) is/are arranged in the direction of the coil axis (SA) relative to the core.

9. Implant (100) according to patent claim 7, wherein
the energy storage (4) comprises at least an energy storage unit (4a) and preferably at least a further energy storage unit (4b); and
the energy storage unit (4a) and, if preferably provided, the further energy storage unit (4b) each have a housing which acts as the field collector (8a) and/or the further field collector (8b).

10. Implant (100) according to any one of patent claims 1 to 9,
wherein
the energy storage (4) comprises at least an energy storage unit and preferably at least a further energy storage unit, and
the field collector (8a) and, if preferably provided, the further field collector (8b) has/have a recess in which the energy storage unit (4a) and, if preferably provided, the further energy storage unit (4b) is/are accommodated.

11. Implant (100) according to any one of patent claims 5 to 10,
wherein
the coil (6) is wound on and around the core (7) between the field collector (8a) and the further field collector (8b).

12. Implant (100) according to any one of patent claims 1 to 7 or 11,
wherein
the energy storage (4) comprises at least an energy storage unit (4a) and preferably at least a further energy storage unit (4b); and
the energy storage unit (4a) and, if preferably provided, the further energy storage unit(s) (4b, 4c, 4d) is/are arranged radially to the coil axis (SA) at least in portions around the coil.

13. Implant (100) according to patent claim 12, wherein
the energy storage (4) completely surrounds the coil axis (SA).

14. Implant (100) according to patent claim 12 or 13, wherein
the energy storage unit (4a, 4b, 4c, 4d) is arranged radially to the coil axis (SA) around the coil (6).

15. Implant (100) according to any one of patent claims 1 to 14, wherein the core (7) and/or the field collector (8a) and/or, if preferably provided, the further field collector (8b) is/are formed from a material with a high relative magnetic permeability and/or a saturation flux density that is as high as possible, for example a ferrite, preferably as a solid material.

16. Implant (100) according to any one of patent claims 1 to 15, wherein the core (7) and/or the field collector (8a) and/or, if preferably provided, the further field collector (8b) has/have a structure formed of a plurality of individual thin layers, and
the material of these layers has a high relative magnetic permeability and/or a high saturation flux density, and is preferably an amorphous metal, such as SiFe, or a Mu metal, such as NiFe.

17. Implant according to patent claim 16, wherein
the field collector (8a) and the further field collector (8b) are an element separate from the core (7) and are formed from a different material, and
the field collector (8a) and the further field collector (8b) are formed from the solid material and the core (7) has the structure with the thin layers.

18. Implant (100) according to any one of the preceding patent claims 1 to 17, wherein the energy receiving portion (5) comprises at least one rectifier (9a) and at least one capacitor (9b) located between the coil (6) and the energy storage (4), and
the coil (6) transfers the received energy to the energy storage (4) via the rectifier (9a) and the capacitor (9b).

19. Implant (100) according to any one of preceding patent claims 1 to 18, wherein
for recharging the energy storage (4), the alternating external magnetic field with a flux density B₀ is to be generated as intended in the area of the implanted implant (100), as a result of which a corresponding charging voltage is induced in the coil (6), which leads to a charging current emitted by the coil (6) and supplied directly or indirectly to the energy storage (4),
the core (7) and/or the field collector (8a) and/or, if preferably provided, the further field collector (8b) is/are formed of a material having a high saturation flux density, and
the geometry of the core (7) and/or of the field collector (8a) and/or, if preferably provided, of the further field collector (8b) is selected such that a flux density B_{C}, which results in the core of the coil (6) from the multiplied flux density (n*B₀) reduced by an opposing field generated by the charging (alternating) current, is in the range of the saturation flux density.

20. Electronic implant (100) according to any one of the preceding patent claims 1 to 19, wherein the implant (100) is an electronic pacemaker, in particular a cardiac pacemaker, and
the electronics assembly (3) connected to the electrode section (2) is configured to monitor the body function via the electrode section (2) and to generate a pulse, in particular a voltage pulse, and to emit this via the electrode section (2) to the body portion for controlling the body function, preferably as a supplement for missing endogenous pulses or for correcting atrophied endogenous pulses, and preferably to measure, store and preferably transmit further body data.

21. Electronic implant (100) according to any one of the preceding claims 1 to 20, wherein an average magnetic flux of 1*10⁻⁹ to 2.5*10⁻⁵ Vs (Weber) is established in the core (7) when using the external alternating magnetic field with a flux density B₀ of 20µT to 10mT.

22. Electronic implant (100) according to any one of the preceding patent claims 1 to 21, wherein
an alternating current resistance (ωL) of the coil (6) resulting from the inductance of the coil (6) and the frequency of the external alternating magnetic field exceeds the ohmic resistance of the coil (6),
the electronics assembly comprises a resonant capacitor, and
alternating current resistance and ohmic resistance for the alternating magnetic field to be used as intended are dimensioned in such a way that the inductance of the coil and the capacitance of the compensation capacitor are in partial resonance or resonance in order to optimize the charging current.

23. Electronic implant (100) according to any one of the preceding patent claims 1 to 22, wherein
the electronics assembly (3) connected to the electrode section (2) is arranged to generate information for the automatic, spatial, preferably parallel, alignment of the coil axis of the charging device to the coil axis of the implant as a condition of implantation of the implant in any spatial alignment in three axes in the body of the living being.

24. Electronic implant (100) according to any one of the preceding patent claims 1 to 23, wherein
the coil (6) is designed such that, in the presence of the alternating magnetic field, it charges the energy store, which preferably has a charge capacity of 200As to 400As, in a time of less than one hour, preferably less than 30 min or 20 min.

25. Electronic implant (100) according to any one of the preceding patent claims 1 to 24, wherein
a cavity is defined between the field collectors, in which the magnetically conductive housing of the energy storage (4) is accommodated as the core (7) or at least as part of the core, in which housing the electrochemical components of the energy storage (4) are accommodated.

26. An electronic implant (100) according to any one of the preceding claims 1 to 25, wherein the energy receiving portion is mirror symmetrical so that the longitudinal magnetic center coincides with the longitudinal center of the coil.

## Revendications

1. Implant électronique (100) destiné à être implanté dans un corps d'un être vivant et à surveiller une fonction corporelle, dans lequel l'implant est un stimulateur cardiaque destiné à surveiller et à commander la fonction corporelle, qui est implanté conformément à sa destination dans/sur le cœur humain, dans lequel l'implant comprend :
une section d'électrode (2) qui, conformément à sa destination, doit être fixée ou disposée sur une partie du corps ; et
un boîtier qui présente un volume V_{G} dans la plage V_{G} ≤ 4 cm³ et qui reçoit les composants suivants de l'implant électronique (100) :
(i) une électronique (3) reliée à la section d'électrode, qui est configurée pour surveiller au moins la fonction corporelle par l'intermédiaire de la section d'électrode (2) ;
(ii) un dispositif de stockage d'énergie (4) destiné à alimenter à long terme l'électronique (3) en énergie électrique, qui peut être rechargé en énergie électrique après décharge ; et
(iii) une section de réception d'énergie (5) reliée électriquement au dispositif de stockage d'énergie (4), qui est configurée de manière à pouvoir recevoir de l'énergie sans contact et la transférer au dispositif de stockage d'énergie (4) pour recharger le dispositif de stockage d'énergie (4) ;
dans lequel
la section de réception d'énergie (5) comprend :
une bobine (6) qui s'étend le long d'un axe de bobine (SA) et qui est configurée pour recevoir l'énergie et la transférer au dispositif de stockage d'énergie (4) lorsqu'elle est traversée par un champ magnétique alternatif externe,
un noyau magnétiquement conducteur (7) qui se trouve dans la bobine (6) et s'étend le long de l'axe de bobine (SA), et
au moins un collecteur de champ (8a) situé à l'extérieur de la bobine (6), qui se trouve, dans la direction de l'axe de bobine (SA), à une extrémité du noyau (7) et présente, transversalement à l'axe de bobine (SA), des dimensions supérieures à celles du noyau (7) ;
dans lequel
le noyau (7), avec l'au moins un collecteur de champ (8a), possède une surface de captation du champ magnétique A₀ perpendiculaire à l'axe de bobine (SA), avec A₀ <=2,5*10⁻³m², qui est définie par A₀ = Φ_{SM}/B₀, où Φ_{SM} est le flux magnétique qui traverse au maximum un milieu longitudinal magnétique situé à l'intérieur de la bobine (6) dans la direction de l'axe de bobine (SA), et B₀ est la densité de flux externe moyenne sur la surface de captation du champ magnétique A₀ ; et
la bobine (6) est conçue de manière à générer, en présence du champ magnétique alternatif, un courant de charge d'une intensité dans une plage de 20mA à 2A.

2. Implant électronique selon la revendication 1, dans lequel
la section de réception d'énergie (5) comprend un redresseur,
la bobine (6) est configurée pour générer, lorsqu'elle est traversée par le champ magnétique alternatif, un courant de charge redressé par le redresseur, qui est fourni au dispositif de stockage d'énergie (4) pour le recharger, et
la bobine (6) est conçue de manière à générer, en présence du champ magnétique alternatif ayant une fréquence dans une plage de 0,5 kHz à 1,5 MHz et du flux magnétique Φ_{SM} dans une plage de 1*10⁻⁹ Vs à 5*10⁻⁵ Vs, le courant de charge d'une intensité dans une plage de 20 mA à 2 A.

3. Implant (100) selon l'une des revendications 1 à 2, dans lequel un nombre de spires W de la bobine (6) se situe dans une plage de W ≤ 2000, de préférence de W ≤ 100.

4. Implant (100) selon l'une des revendications 1 à 3, où
le collecteur de champ (8a) est une partie du noyau (7), en particulier est formé de manière monolithique avec le noyau (7), à partir du même matériau.

5. Implant (100) selon l'une des revendications 1 à 4, comprenant en outre :
un autre collecteur de champ (8b), qui se trouve, dans la direction de l'axe de bobine (SA), à une autre extrémité du noyau (7) et présente, transversalement à l'axe de bobine (SA), des dimensions supérieures à celles du noyau (7).

6. Implant (100) selon la revendication 5, où
l'autre collecteur de champ (8b) est une partie du noyau (7), en particulier est formé de manière monolithique avec le noyau (7), à partir du même matériau.

7. Implant (100) selon l'une des revendications 1 ou 5, dans lequel
le collecteur de champ (8a) et/ou l'autre collecteur de champ (8b) est un élément distinct du noyau (7) et de préférence formé à partir d'un autre matériau.

8. Implant (100) selon l'une des revendications 1 à 7, où
le dispositif de stockage d'énergie (4) comprend au moins une unité de stockage d'énergie (4a) et de préférence au moins une autre unité de stockage d'énergie (4b) ; et
l'une des unités de stockage d'énergie (4a) et, si elle est prévue de préférence, l'autre unité de stockage d'énergie (4b) est/sont disposée/disposées dans la direction de l'axe de bobine (SA) par rapport au noyau.

9. Implant (100) selon la revendication 7, où
le dispositif de stockage d'énergie (4) comprend au moins une unité de stockage d'énergie (4a) et de préférence au moins une autre unité de stockage d'énergie (4b) ; et
l'une des unités de stockage d'énergie (4a) et, si elle est prévue de préférence, l'autre unité de stockage d'énergie (4b) comprend/comprennent chacune un boîtier qui agit/agissent comme le collecteur de champ (8a) et/ou l'autre collecteur de champ (8b).

10. Implant (100) selon l'une des revendications 1 à 9, où
le dispositif de stockage d'énergie (4) comprend au moins une unité de stockage d'énergie et de préférence au moins une autre unité de stockage d'énergie, et
le collecteur de champ (8a) et, s'il est prévu de préférence, l'autre collecteur de champ (8b) présente/présentent un creux dans lequel/lesquels l'une des unités de stockage d'énergie (4a) et, si elle est prévue de préférence, l'autre unité de stockage d'énergie (4b) est/sont reçue/reçues.

11. Implant (100) selon l'une des revendications 5 à 10, où
la bobine (6) est enroulée sur et autour du noyau (7), entre le collecteur de champ (8a) et l'autre collecteur de champ (8b).

12. Implant (100) selon l'une des revendications 1 à 7 ou 11, dans lequel
le dispositif de stockage d'énergie (4) comprend au moins une unité de stockage d'énergie (4a) et de préférence au moins une autre unité de stockage d'énergie (4b) ; et
l'une des unités de stockage d'énergie (4a) et, si elle est/elles sont prévue/prévues de préférence, l'autre/les autres unité/unités de stockage d'énergie (4b, 4c, 4d) est/sont disposée/disposées radialement par rapport à l'axe de bobine (SA), au moins par sections autour de la bobine.

13. Implant (100) selon la revendication 12, où
le dispositif de stockage d'énergie (4) entoure complètement l'axe de bobine (SA).

14. Implant (100) selon la revendication 12 ou 13, dans lequel
l'unité de stockage d'énergie (4a, 4b, 4c, 4d) est disposée radialement par rapport à l'axe de bobine (SA) autour de la bobine (6).

15. Implant (100) selon l'une des revendications 1 à 14, dans lequel le noyau (7) et/ou le collecteur de champ (8a) et/ou, s'il est prévu de préférence, l'autre collecteur de champ (8b) est/sont formé/formés à partir d'un matériau ayant une perméabilité magnétique relative élevée et/ou une densité de flux à saturation aussi élevée que possible, par exemple une ferrite, de préférence sous forme de matériau massif.

16. Implant (100) selon l'une des revendications 1 à 15, dans lequel le noyau (7) et/ou le collecteur de champ (8a) et/ou, s'il est prévu de préférence, l'autre collecteur de champ (8b) présente/présentent une structure formée d'une pluralité de couches minces individuelles, et
le matériau de ces couches présente une perméabilité magnétique relative élevée et/ou une densité de flux à saturation élevée, et est de préférence un métal amorphe, par exemple SiFe, ou un mu-métal, tel que NiFe.

17. Implant selon la revendication 16, dans lequel
le collecteur de champ (8a) et l'autre collecteur de champ (8b) est un élément distinct du noyau (7) et formé à partir d'un autre matériau, et
le collecteur de champ (8a) et l'autre collecteur de champ (8b) sont formés à partir du matériau massif et le noyau (7) présente la structure à couches minces.

18. Implant (100) selon l'une des revendications précédentes 1 à 17, dans lequel la section de réception d'énergie (5) comprend au moins un redresseur (9a) et au moins un condensateur (9b), qui se trouvent entre la bobine (6) et le dispositif de stockage d'énergie (4), et
la bobine (6) transfère l'énergie reçue au dispositif de stockage d'énergie (4) par l'intermédiaire du redresseur (9a) et du condensateur (9b).

19. Implant (100) selon l'une des revendications précédentes 1 à 18, dans lequel
pour recharger le dispositif de stockage d'énergie (4), le champ magnétique alternatif externe ayant une densité de flux B₀ doit être généré, conformément à sa destination, dans la zone de l'implant (100) implanté, ce qui induit une tension de charge correspondante dans la bobine (6), laquelle produit un courant de charge fourni par la bobine (6) et acheminé indirectement ou directement au dispositif de stockage d'énergie (4),
le noyau (7) et/ou le collecteur de champ (8a) et/ou, s'il est prévu de préférence, l'autre collecteur de champ (8b) est/sont formé/formés à partir d'un matériau qui présente une densité de flux à saturation élevée, et
la géométrie du noyau (7) et/ou du collecteur de champ (8a) et/ou, s'il est prévu de préférence, de l'autre collecteur de champ (8b) est choisie de manière à ce qu'une densité de flux B_{K}, qui résulte dans le noyau de la bobine (6) de la densité de flux multipliée (n*B₀), diminuée d'un champ opposé généré par le courant (alternatif) de charge, se situe dans la plage de la densité de flux à saturation.

20. Implant électronique (100) selon l'une des revendications précédentes 1 à 19, dans lequel l'implant (100) est un stimulateur électronique, en particulier un stimulateur cardiaque, et
l'électronique (3) reliée à la section d'électrode (2) est configurée pour surveiller la fonction corporelle par l'intermédiaire de la section d'électrode (2) et pour générer une impulsion, en particulier une impulsion de tension, et la transmettre par l'intermédiaire de la section d'électrode (2) à la partie du corps afin de commander la fonction corporelle, de préférence pour remplacer des impulsions endogènes manquantes ou pour corriger des impulsions endogènes affaiblies, et de préférence pour mesurer, mémoriser et de préférence envoyer d'autres données corporelles.

21. Implant électronique (100) selon l'une des revendications précédentes 1 à 20, dans lequel, lors de l'utilisation du champ magnétique alternatif externe ayant une densité de flux B₀ de 20 µT à 10 mT, un flux magnétique moyen de 1*10⁻⁹ à 2,5*10⁻⁵ Vs (weber) s'établit dans le noyau (7).

22. Implant électronique (100) selon l'une des revendications précédentes 1 à 21, dans lequel
une réactance (ωL) de la bobine (6), résultant de l'inductance de la bobine (6) et de la fréquence du champ magnétique alternatif externe, dépasse la résistance ohmique de la bobine (6),
l'électronique possède un condensateur de compensation, et
la réactance et la résistance ohmique sont dimensionnées pour le champ magnétique alternatif à utiliser conformément à sa destination de manière à ce que, pour optimiser le courant de charge, l'inductance de la bobine et la capacité du condensateur de compensation soient en résonance partielle ou en résonance.

23. Implant électronique (100) selon l'une des revendications précédentes 1 à 22, dans lequel
l'électronique (3) reliée à la section d'électrode (2) est configurée pour générer des informations permettant l'alignement spatial automatique, de préférence parallèle, de l'axe de bobine du chargeur par rapport à l'axe de bobine de l'implant, comme condition de l'implantation de l'implant dans le corps de l'être vivant selon une orientation spatiale quelconque suivant trois axes.

24. Implant électronique (100) selon l'une des revendications précédentes 1 à 23, dans lequel
la bobine (6) est conçue de manière à charger, en présence du champ magnétique alternatif, le dispositif de stockage d'énergie, qui possède de préférence une capacité de charge de 200 As à 400 As, en moins d'une heure, de préférence en moins de 30 min ou de 20 min.

25. Implant électronique (100) selon l'une des revendications précédentes 1 à 24, dans lequel
une cavité est définie entre les collecteurs de champ, dans laquelle le boîtier magnétiquement conducteur du dispositif de stockage d'énergie (4) est reçu en tant que noyau (7) ou au moins en tant que partie du noyau, dans lequel sont reçus les composants électrochimiques du dispositif de stockage d'énergie (4).

26. Implant électronique (100) selon l'une des revendications précédentes 1 à 25, dans lequel la section de réception d'énergie présente une structure à symétrie miroir, de sorte que le milieu longitudinal magnétique coïncide avec le milieu longitudinal de la bobine.
